(19) Europäisches Patentamt
European Patent Office
Office européen des brevets

(11) Veröffentlichungsnummer : **0 007 990**
**B1**

(12) # EUROPÄISCHE PATENTSCHRIFT

(45) Veröffentlichungstag der Patentschrift : **25.03.81**

(51) Int. Cl.³ : **C 07 D231/20**, C 07 D231/22, C 07 D231/18, A 01 N 43/56

(21) Anmeldenummer : **79102055.5**

(22) Anmeldetag : **21.06.79**

(54) **Pyrazolätherderivate, Verfahren zu ihrer Herstellung und Herbizide, die diese Verbindungen enthalten.**

(30) Priorität : **04.07.78 DE 2829289**

(43) Veröffentlichungstag der Anmeldung : **20.02.80 (Patentblatt 80/04)**

(45) Bekanntmachung des Hinweises auf die Patenterteilung : **25.03.81 Patentblatt 81/12**

(84) Benannte Vertragsstaaten : **AT BE CH DE FR GB IT LU NL SE**

(56) Entgegenhaltungen :
**US - A - 3 303 200**
**US - A - 3 822 283**
**CHEMICAL ABSTRACTS, Vol. 85, Nr. 1, 5. Juli 1976, Seite 454, Nr. 5620s Columbus, Ohio, USA**
**CHEMICAL ABSTRACTS, Vol. 85, Nr. 7, 16. August 1976, Seite 523, Nr. 46637w Columbus, Ohio, USA**

(73) Patentinhaber : **BASF Aktiengesellschaft**
**Carl-Bosch-Strasse 38**
**D-6700 Ludwigshafen (DE)**

(72) Erfinder : **Plath, Peter, Dr.-Chem.**
**Berner Weg 24**
**D-6700 Ludwigshafen (DE)**
Erfinder : **Rohr, Wolfgang, Dr.-Chem.**
**Gontardstrasse 4**
**D-6800 Mannheim 1 (DE)**
Erfinder : **Wuerzer, Bruno, Dr.**
**Wilhelm-Busch-Strasse 55**
**D-6703 Limburgerhof (DE)**
Erfinder : **Becker, Rainer, Dr.**
**Sonnenwendstrasse 83 a**
**D-6702 Bad Duerkheim 1 (DE)**

**EP 0 007 990 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Pyrazolätherderivate, Verfahren zu ihrer Herstellung und Herbizide, die diese Verbindungen enthalten

Die vorliegende Erfindung betrifft wertvolle neue substituierte Pyrazolätherderivate und deren Salze mit herbizider Wirkung sowie Herbizide, die diese Verbindungen als Wirkstoff enthalten, und Verfahren zur Bekämpfung unerwünschten Pflanzenwuchses mit diesen Verbindungen.

Es ist bekannt, substituierte Pyrazole oder Pyrazoliumsalze, z.B. das 1,2-Dimethyl-3,5-diphenylpyrazolium-methyl-sulfat, als Herbizide zu verwenden (DE-OS 2 513 750, DE-OS 2 260 485). Ferner wird das bekannte 3-Isopropyl-2,1,3-benzothidiazin-4-on-2,2-dioxid (DE-PS 1 542 836) in der Praxis in großen Mengen als Herbizid verwendet.

Es wurde nun gefunden, daß Pyrazolätherderivate der Formel

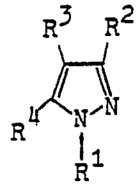

(1)

in welcher $R^1$ Wasserstoff oder $-\underset{O}{\underset{\|}{C}}-R^5$ bedeutet, wobei

$R^5$ Wasserstoff, Alkoxyalkyl, Alkyl oder Halogenalkyl bedeutet,

$R^1$ ferner $-\underset{O}{\underset{\|}{C}}-O-R^6$ bedeutet, wobei

$R^6$ Alkyl oder Phenyl bedeutet, das gegebenenfalls ein-oder mehrfach durch Halogen substituiert ist,

$R^2$ die Gruppe $Y-R^7$ bedeutet, wobei Y Sauerstoff oder Schwefel bedeutet und $R^7$ einen aliphatischen, cycloaliphatischen oder araliphatischen Kohlenwasserstoffrest, Cyclopentylmethyl oder einen Arylrest bedeutet, wobei dieser Arylrest gegebenenfalls ein- oder mehrfach durch Alkyl, Halogen, Halogenalkyl, Alkoxy substituiert ist,

$R^3$ den Methoxycarbonylrest bedeutet und

$R^4$ Wasserstoff oder Methyl bedeutet und die Salze der Pyrazolätherderivate eine gute herbizide und gegenüber Kulturpflanzen selektive herbizide Wirkung zeigen.

Salze sind die Salze mit anorganischen oder organischen Säuren, z.B. Salzsäure, Orthophosphorsäure, Schwefelsäure, Ameisensäure, Trichloressigsäure, Methansulfonsäure, p-Toluolsulfonsäure oder Dodecylbenzolsulfonsäure.

Die neuen Pyrazolätherderivate liegen meist als Isomere vor.

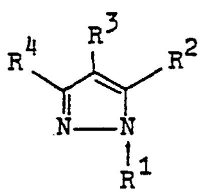

und

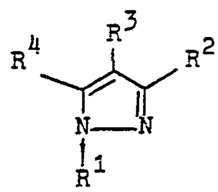

Das Isomerenverhältnis wird im wesentlichen durch die verschiedenen Substituenten bestimmt.

Solange nicht besonders erwähnt wird, daß lediglich eines der beiden Isomeren vorliegt, soll im folgenden unter einer bestimmten Formel oder Bezeichnung stets das Isomerengemisch verstanden werden.

In der allgemeinen Formel bedeutet $R^1$ beispielsweise Wasserstoff oder $-\underset{O}{\underset{\|}{C}}-R^5$ oder $-\underset{O}{\underset{\|}{C}}-O-R^6$, wobei $R^5$ beispielsweise geradkettiges oder verzweigtes Alkyl mit 1 bis 16 Kohlenstoffatomen, das gegebenenfalls durch Halogen oder Alkoxy mit 1 bis 4 Kohlenstoffatomen substituiert ist, bedeutet.

$R^6$ bedeutet beispielsweise geradkettiges oder verzweigtes Alkyl mit 1 bis 16 Kohlenstoffatomen oder gegebenenfalls ein- oder mehrfach durch Fluor, Chlor, Brom substituiertes Phenyl.

$R^2$ bedeutet die Gruppe $-Y-R^7$, wobei Y Sauerstoff oder Schwefel bedeutet und $R^7$ beispielsweise geradkettiges oder verzweigtes Alkyl mit 1 bis 18 Kohlenstoffatomen bedeutet.

$R^7$ bedeutet außerdem geradkettiges oder verzweigtes Alkenyl mit 3 bis 18 Kohlenstoffatomen oder Cycloalkyl mit 3 bis 8 Kohlenstoffatomen.

$R^7$ bedeutet ferner beispielsweise Arylalkyl mit 1 bis 3 Kohlenstoffatomen im Arylteil oder $R^7$ bedeutet gegebenenfalls ein- oder mehrfach durch Fluor, Chlor, Brom, Trifluormethyl, Alkyl, Alkoxy — jeweils mit 1 bis 4 Kohlenstoffatomen — substituiertes Aryl mit 6 bis 10 Kohlenstoffatomen.

Nachfolgend wird die Herstellung der neuen Pyrazolätherderivate näher beschrieben.

Die Herstellung kann entsprechend literaturbekannten Verfahren, z.B. Ark. Kemi *4,* 297-323 (1952), Ark. Kemi *8,* 523-544 (1955), Chem. Ber. *92,* 2593 (1959) vorgenommen werden.

Die Umsetzung läßt, sich für den Fall, daß $R^1$ = Wasserstoff, $R^2$ = Methoxy, $R^3$ = Methoxycarbonyl und $R^4$ = Methyl bedeutet, durch das Formelschema (2) beschreiben :

$$CH_3\text{-O-}\overset{\overset{S}{\|}}{C}\text{-NH-NH}_2 + CH_3\text{-CO-CHCl-CO}_2CH_3 \qquad (2)$$

(A) (B)

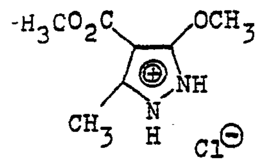

+ S

Das als Hydrochlorid anfallende Pyrazolätherderivat wird nach bekannten Methoden neutralisiert und durch Extraktion oder Umkristallisation vom elementaren Schwefel abgetrennt. Das als Zwischenprodukt benötigte Thiokohlensäure-alkylesterhydrazid (A) läßt sich nach bakannten Methoden herstellungen, z.B. Acta chem. Scand. *23,* 1916-1934 (1969).

Ebenso ist die Herstellung von 2-Chlor-1,3-dicarbonylverbindungen wie (B) bekannt, z.B. durch Umsetzung der β-Dicarbonylverbindung mit Sulfurylchlorid.

Die nachstehenden Beispiele dienen zur Erläuterung des Herstellungsverfahrens :

## Beispiel 1

3-Benzylthio-5-methyl-4-methoxycarbonyl-pyrazol

Zu einer Lösung von 55 g Dithiocarbazinsäure-S-benzyl-ester in 250 ml Tetrahydrofuran gibt man bei Raumtemperatur 47 g 2-Chloracetessigsäuremethylester. Nach 16 Stunden Rühren bei Raumtemperatur saugt man vom ausgefallenen Feststoffgemisch ab, wäscht mit Diäthyläther nach und rührt den Rückstand dann in 200 ml 12-proz. Ammoniaklösung ein. Das Produkt wird durch Extraktion mit Methylenchlorid vom Schwefel abgetrennt. Nach Trocknung über Natriumsulfat wird das Methylenchlorid abdestilliert und der verbleibende Feststoff aus Essigester umkristallisiert. Fp. 109-110° C.

## Beispiel 2

Acetat von 3-(2',3'-Dimethylphenoxy)-5-methyl-4-methoxycarbonyl-pyrazol

Man gibt 15 g 3-(2'-3'-Dimethylphenoxy)-5-methyl-4-methoxycarbonyl-pyrazol zu 25 g Acetanhydrid und erhitzt das Reaktionsgemisch 5 Minuten zum Sieden. Nach dem Abkühlen wird mit 150 ml Wasser versetzt und 30 Minuten kräftig gerührt. Nach Absaugen und Trocknen erhält man in nahezu quantitativer Ausbeute einen weißen Feststoff mit Fp. 111° C.

## Beispiel 3

1,4-Bis(methoxycarbonyl)-3-(2',3'-dimethylphenoxy)-5-methylpyrazol

Man stellt eine Mischung aus 5,6 g Triäthylamin und 13 g 3-(2',3'-Dimethylphenoxy)-5-methyl-4-methoxycarbonyl-pyrazol und 100 ml Tetrahydrofuran her und tropft unter Rühren und Kühlung 4,9 g Chlorkohlensäuremethylester zu. Nach 16-stündigem Rühren wird vom ausgefallenen Hydrochlorid abgesaugt. Das Filtrat wird eingeengt und der verbleibende Rückstand anschließend aus Essigester umkristallisiert. Fp. 115° C.

In entsprechender Weise wurden die folgenden Substanzen erhalten :

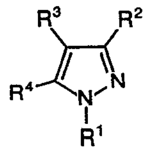

| Nr. | $R^1$ | $R^2$ | $R^3$ | $R^4$ | Fp. (°C) |
|---|---|---|---|---|---|
| 5 | Wasserstoff | $—O—CH_3$ | $—CO_2CH_3$ | $CH_3$ | |
| 6 | Wasserstoff | $—O—C_2H_5$ | $—CO_2CH_3$ | $CH_3$ | |
| 7 | Wasserstoff | $—O—C_3H_7—i$ | $—CO_2CH_3$ | $CH_3$ | 125-126 |
| 8 | Wasserstoff | $—O—C_4H_9—n$ | $—CO_2CH_3$ | $CH_3$ | |
| 9 | Wasserstoff | $—O\,C_4H_9—$sek. | $—CO_2CH_3$ | $CH_3$ | 65-67 |
| 10 | Wasserstoff | $—O—C_4H_9—$iso | $—CO_2CH_3$ | $CH_3$ | 86 |
| 11 | Wasserstoff | $—O—C_4H_9—$tert. | $—CO_2CH_3$ | $CH_3$ | |
| 12 | Acetyl | $—O—C_4H_9—$sek. | $—CO_2CH_3$ | $CH_3$ | 83 |
| 13 | Chloracetyl | $—O—C_4H_9—$sek. | $—CO_2CH_3$ | $CH_3$ | |
| 14 | Dichloracetyl | $—O—C_4H_9—$sek. | $—CO_2CH_3$ | $CH_3$ | |
| 15 | Acetyl | $—O—C_3H_7—i$ | $—CO_2CH_3$ | $CH_3$ | 58-59 |
| 16 | Propionyl | $—O—C_3H_7—i$ | $—CO_2CH_3$ | $CH_3$ | |
| 17 | Methoxyacetyl | $—O—C_3H_7—i$ | $—CO_2CH_3$ | $CH_3$ | |
| 21 | Methoxycarbonyl | $—O—C_4H_9—$iso | $—CO_2CH_3$ | $CH_3$ | |
| 22 | Isopropoxycarbonyl | $—O—C_4H_9—$iso | $—CO_2CH_3$ | $CH_3$ | |
| 23 | Phenoxycarbonyl | $—O—C_4H_9—$iso | $—CO_2CH_3$ | $CH_3$ | 104-105 |
| 40 | Wasserstoff | —O— 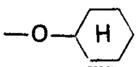 | $—CO_2CH_3$ | $CH_3$ | 123 |
| 41 | Acetyl | —O— 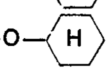 | $—CO_2CH_3$ | $CH_3$ | 112-114 |
| 42 | Phenoxycarbonyl | —O— 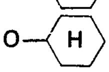 | $—CO_2CH_3$ | $CH_3$ | 104-106 |
| 43 | 3′-Chlorphenoxycarbonyl | —O— 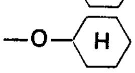 | $—CO_2CH_3$ | $CH_3$ | |
| 44 | Wasserstoff | $—S—C_3H_7—i$ | $—CO_2CH_3$ | $CH_3$ | 117-119 |
| 45 | Phenoxycarbonyl | $—S—C_3H_7—i$ | $—CO_2CH_3$ | $CH_3$ | 86-89 |
| 46 | 3′-Chlorphenoxycarbonyl | $—S—C_3H_7—i$ | $—CO_2CH_3$ | $CH_3$ | |
| 49 | Wasserstoff | Cyclopentylmethyl-oxy | $—CO_2CH_3$ | $CH_3$ | 84 |
| 50 | H | 2-Pentyloxy | $—CO_2CH_3$ | $CH_3$ | 01,$n_D^{23}$ = 1,4960 |
| 51 | Acetyl | Cyclopentylmethyl-oxy | $—CO_2CH_3$ | $CH_3$ | 75-76 |
| 52 | Wasserstoff | $—O—\underset{\displaystyle CH_3}{\underset{\vert}{CH}}—CH_2—C_3H_7—i$ | $—CO_2CH_3$ | $CH_3$ | 81 |
| 53 | Wasserstoff | $—O—CH(C_2H_5)_2$ | $—CO_2CH_3$ | $CH_3$ | 80-82 |
| 54 | Wasserstoff | $—O—CH\genfrac{}{}{0pt}{}{\diagup C_2H_5}{\diagdown C_3H_7—n}$ | $—CO_2CH_3$ | $CH_3$ | 01,$n_D^{26}$ = 1,4982 |
| 55 | Wasserstoff | $—O—CH(n—C_3H_7)_2$ | $—CO_2CH_3$ | $CH_3$ | 61 |
| 56 | Wasserstoff | $—O—CH(C_3H_7—i)_2$ | $—CO_2CH_3$ | $CH_3$ | 82 |
| 63 | Acetyl | $—O—CH_2—C_3H_7—i$ | $—CO_2CH_3$ | $CH_3$ | 72 |
| 66 | Wasserstoff | $—O—CH_2—C_4H_9—$tert. | $—CO_2CH_3$ | $CH_3$ | 66-68 |
| 67 | Acetyl | $—O—CH_2—C_4H_9—$tert. | $—CO_2CH_3$ | $CH_3$ | 98-99 |
| 68 | Phenoxy-carbonyl | $—O—CH_2—C_4H_9—$tert. | $—CO_2CH_3$ | $CH_3$ | 128-129 |
| 69 | Wasserstoff | $—O—CH_2—\underset{\displaystyle CH_3}{\underset{\vert}{CH}}—C_2H_5$ | $—CO_2CH_3$ | $CH_3$ | 46 |
| 70 | Wasserstoff | $—O—CH_2—CH(C_2H_5)_2$ | $—CO_2CH_3$ | $CH_3$ | 47-48 |
| 71 | Wasserstoff | $—O—CH_2—\underset{\displaystyle CH_3}{\underset{\vert}{CH}}—CH_2C_3H_7—i$ | $—CO_2CH_3$ | $CH_3$ | $n_D^{25}$ = 1,5469 |

| Nr. | R¹ | R² | R³ | R⁴ | Fp. (°C) |
|---|---|---|---|---|---|
| 72 | Wasserstoff | 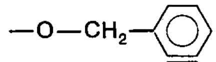 | $-CO_2CH_3$ | $CH_3$ | |
| 73 | Wasserstoff | 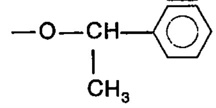 | $-CO_2CH_3$ | $CH_3$ | |
| 74 | Wasserstoff | 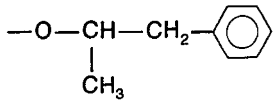 | $-CO_2CH_3$ | $CH_3$ | $n_D^{27} = 1{,}5459$ |
| 75 | Wasserstoff | 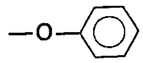 | $-CO_2CH_3$ | $CH_3$ | 109-110 |
| 76 | Acetyl | 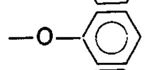 | $-CO_2CH_3$ | $CH_3$ | 68-70 |
| 77 | Phenoxycarbonyl | 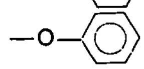 | $-CO_2CH_3$ | $CH_3$ | |
| 78 | Wasserstoff | 2′-Methyl-phenoxy | $-CO_2CH_3$ | $CH_3$ | 123 |
| 79 | Acetyl | 2′-Methyl-phenoxy | $-CO_2CH_3$ | $CH_3$ | 91 |
| 80 | Phenoxycarbonyl | 2′-Methyl-phenoxy | $-CO_2CH_3$ | $CH_3$ | |
| 81 | Wasserstoff | 2′,3′-Dimethylphenoxy | $-CO_2CH_3$ | $CH_3$ | 150 |
| 82 | Wasserstoff | 2′,4′-Dimethylphenoxy | $-CO_2CH_3$ | $CH_3$ | 161-163 |
| 83 | Acetyl | 2′,4′-Dimethylphenoxy | $-CO_2CH_3$ | $CH_3$ | 81-82 |
| 84 | Wasserstoff | 2′,5′-Dimethylphenoxy | $-CO_2CH_3$ | $CH_3$ | 100-102 |
| 85 | Acetyl | 2′,5′-Dimethylphenoxy | $-CO_2CH_3$ | $CH_3$ | 109-110 |
| 86 | Wasserstoff | 3′-Methyl-4′-chlorphenoxy | $-CO_2CH_3$ | $CH_3$ | 148 |
| 87 | Wasserstoff | 2′-Methyl-4′-chlorphenoxy | $-CO_2CH_3$ | $CH_3$ | 146-147 |
| 88 | Wasserstoff | 4′-Methylphenoxy | $-CO_2CH_3$ | $CH_3$ | 137-139 |
| 89 | Wasserstoff | 4′-Chlorphenoxy | $-CO_2CH_3$ | $CH_3$ | 106 |
| 90 | Wasserstoff | 3′-Methoxyphenoxy | $-CO_2CH_3$ | $CH_3$ | 109 |
| 91 | Acetyl | 3′-Methoxyphenoxy | $-CO_2CH_3$ | $CH_3$ | 70-71 |
| 92 | Wasserstoff | 3′-Isopropyl-Phenoxy | $-CO_2CH_3$ | $CH_3$ | 72 |
| 95 | Wasserstoff | 4′-Fluorphenoxy | $-CO_2CH_3$ | $CH_3$ | |
| 102 | Wasserstoff | Cyclopentyloxy | $-CO_2CH_3$ | $-CH_3$ | |
| 103 | Wasserstoff | Cyclopentylthio | $-CO_2CH_3$ | $-CH_3$ | 92 |
| 104 | Wasserstoff | Cyclooctyloxy | $-CO_2CH_3$ | $-CH_3$ | |
| 105 | Wasserstoff | Cyclooctylthio | $-CO_2CH_3$ | $-CH_3$ | |
| 106 | Wasserstoff | Cyclohexylthio | $-CO_2CH_3$ | $-CH_3$ | 113-114 |
| 121 | Wasserstoff | 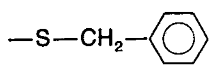 | $-CO_2CH_3$ | $-CH_3$ | 109-110 |
| 122 | Wasserstoff | 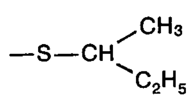 | $-CO_2CH_3$ | $-CH_3$ | 53 |
| 123 | Wasserstoff | $-S-CH_2-C_3H_7-i$ | $-CO_2CH_3$ | $-CH_3$ | 92-94 |
| 124 | Wasserstoff | $-S-(CH_2)_2-C_3H_7-i$ | $-CO_2CH_3$ | $-CH_3$ | 83-84 |
| 125 | Wasserstoff | 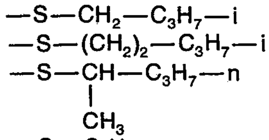 | $-CO_2CH_3$ | $-CH_3$ | 54 |
| 126 | Wasserstoff | $-S-C_7H_{15}-n$ | $-CO_2CH_3$ | $-CH_3$ | 58-60 |
| 127 | Wasserstoff | $-S-C_8H_{17}-n$ | $-CO_2CH_3$ | $-CH_3$ | 54-55 |
| 128 | Wasserstoff | 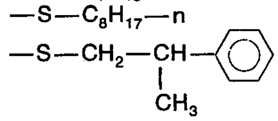 | $-CO_2CH_3$ | $-CH_3$ | 100-101 |
| 129 | Wasserstoff | 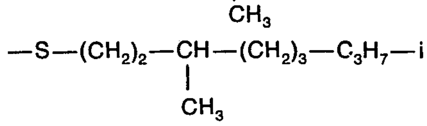 | $-CO_2CH_3$ | $-CH_3$ | $n_D^{26} = 1{,}5195$ |
| 132 | Wasserstoff | Thiophenyl | $-CO_2CH_3$ | $-CH_3$ | 109-110 |
| 133 | Wasserstoff | 2′-Methylphenyl-thio | $-CO_2CH_3$ | $-CH_3$ | |
| 134 | Wasserstoff | 3′-Methylphenyl-thio | $-CO_2CH_3$ | $-CH_3$ | |
| 135 | Wasserstoff | 2′-Methoxyphenyl-thio | $-CO_2CH_3$ | $-CH_3$ | |

| Nr. | $R^1$ | $R^2$ | $R^3$ | $R^4$ | Fp. (°C) |
|---|---|---|---|---|---|
| 137 | Wasserstoff | 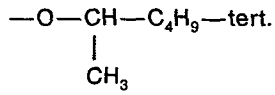 | $CO_2CH_3$ | $CH_3$ | 88-91 |
| 161 | Wasserstoff | $—O—C_4H_9—iso$ | $CO_2CH_3$ | Wasserstoff | |
| 162 | Acetyl | $—O—C_4H_9—iso$ | $CO_2CH_3$ | Wasserstoff | |
| 163 | Phenoxycarbonyl | $—O—C_4H_9—iso$ | $CO_2CH_3$ | Wasserstoff | |
| 168 | Acetyl | 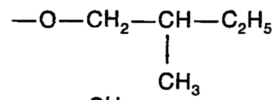 | $CO_2CH_3$ | $—CH_3$ | 46 |
| 169 | Acetyl | 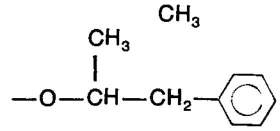 | $CO_2CH_3$ | $—CH_3$ | $n_D^{25} = 1{,}5412$ |
| 170 | Acetyl | $—O—CH(C_2H_5)_2$ | $CO_2CH_3$ | $—CH_3$ | 41 |
| 172 | Acetyl | Thiobenzyl | $CO_2CH_3$ | $—CH_3$ | 92 |
| 173 | Phenoxycarbonyl | Thiobenzyl | $CO_2CH_3$ | $—CH_3$ | 105-107 |
| 174 | Acetyl | $—S—C_7H_{15}—n$ | $CO_2CH_3$ | $—CH_3$ | 55-57 |
| 175 | Acetyl | $—S—C_8H_{17}—n$ | $CO_2CH_3$ | $—CH_3$ | 38-40 |
| 176 | Acetyl | 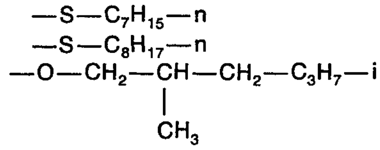 | $CO_2CH_3$ | $—CH_3$ | $n_D^{26} = 1{,}4875$ |
| 177 | Acetyl | 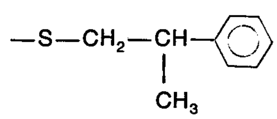 | $CO_2CH_3$ | $—CH_3$ | 67-68 |
| 179 | Phenoxycarbonyl | $—S—CH_2—C_3H_7—i$ | $—CO_2CH_3$ | $—CH_3$ | 95-96 |
| 180 | Wasserstoff | 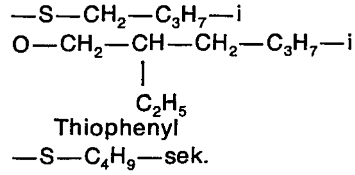 | $—CO_2CH_3$ | $—CH_3$ | $n_D^{26} = 1{,}4912$ |
| 181 | Acetyl | Thiophenyl | $CO_2CH_3$ | $—CH_3$ | 86-88 |
| 182 | Acetyl | $—S—C_4H_9—sek.$ | $CO_2CH_3$ | $CH_3$ | 76-78 |
| 183 | Acetyl | 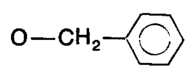 | $CO_2CH_3$ | $CH_3$ | |
| 186 | Phenoxycarbonyl | 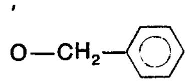 | $CO_2CH_3$ | $CH_3$ | |
| 187 | Phenoxycarbonyl | Thiophenyl | $CO_2CH_3$ | $CH_3$ | 119-121 |
| 188 | Phenoxycarbonyl | $—O—CH_2—CH(C_2H_5)_2$ | $CO_2CH_3$ | $CH_3$ | 87-88 |
| 189 | Phenoxycarbonyl | $—O—C_3H_7—i$ | $CO_2CH_3$ | $CH_3$ | 75-77 |
| 190 | Phenoxycarbonyl | 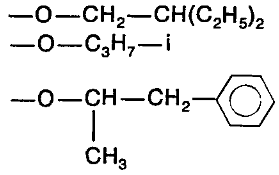 | $CO_2CH_3$ | $CH_3$ | 74-75 |
| 191 | Phenoxycarbonyl | 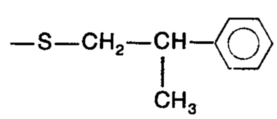 | $CO_2CH_3$ | $CH_3$ | 90-91 |
| 192 | Wasserstoff | 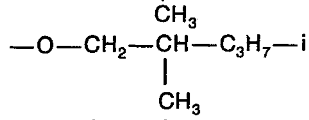 | $CO_2CH_3$ | $CH_3$ | $n_D^{27} = 1{,}4902$ |
| 193 | Acetyl | 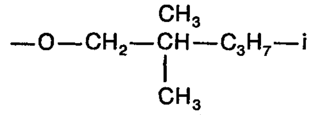 | $CO_2CH_3$ | $CH_3$ | 72-73 |
| 194 | Phenoxycarbonyl | 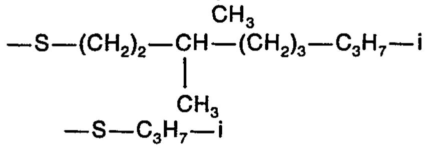 | $CO_2CH_3$ | $CH_3$ | $n_D^{30} = 1{,}5318$ |
| 195 | Methoxycarbonyl | $—S—C_3H_7—i$ | $CO_2CH_3$ | $CH_3$ | 87-88 |

| Nr. | $R^1$ | $R^2$ | $R^3$ | $R^4$ | Fp. (°C) |
|---|---|---|---|---|---|
| 196 | 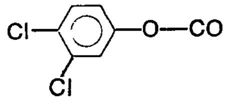 | 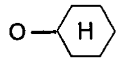 | $-CO_2CH_3$ | $-CH_3$ | 106-108 |
| 197 | 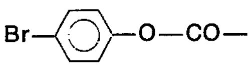 | 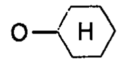 | $-CO_2CH_3$ | $-CH_3$ | 61-63 |
| 199 | $HC\equiv C-CH_2-O-CO-$ | $-O-C_4H_9-$iso | $-CO_2CH_3$ | $-CH_3$ | |
| 200 | n$-C_3H_7-O-CO-$ | $-O-C_4H_9-$iso | $-CO_2CH_3$ | $-CH_3$ | |
| 201 | n$-C_4H_9-O-CO-$ | $-O-C_4H_9-$iso | $-CO_2CH_3$ | $-CH_3$ | 51 |
| 211 | 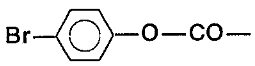 | $-O-C_4H_9-$iso | $-CO_2CH_3$ | $-CH_3$ | 142 |
| 212 | 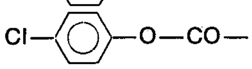 | $-O-C_4H_9-$iso | $-CO_2CH_3$ | $-CH_3$ | 75 |
| 214 | 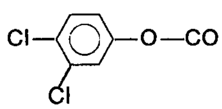 | $-O-C_4H_9-$iso | $-CO_2CH_3$ | $-CH_3$ | 96-97 |
| 216 | 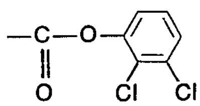 | 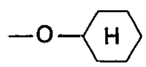 | $-CO_2CH_3$ | $-CH_3$ | 112-113 |
| 217 | 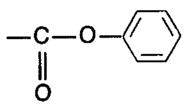 | 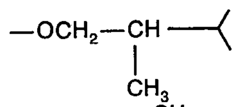 | $-CO_2CH_3$ | $-CH_3$ | 84 |
| 218 | 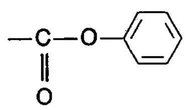 | 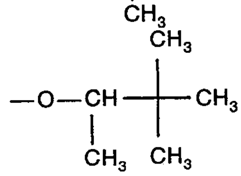 | $-CO_2CH_3$ | $-CH_3$ | 86-88 |
| 219 | 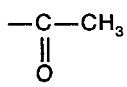 | 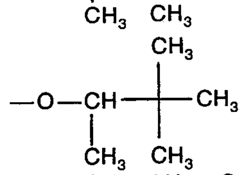 | $-CO_2CH_3$ | $-CH_3$ | 102-103 |
| 220 | H | 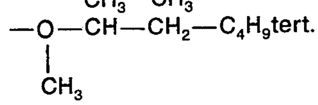 | $-CO_2CH_3$ | $-CH_3$ | 157-158 |
| 221 | 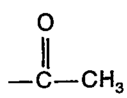 | 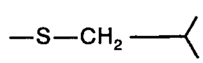 | $-CO_2CH_3$ | $-CH_3$ | 40-41 |
| 223 | 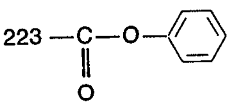 | 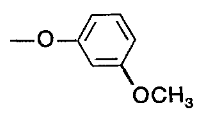 | $-CO_2CH_3$ | $-CH_3$ | 105-107 |
| 228 | 3′-Chlorphenoxycarbonyl | 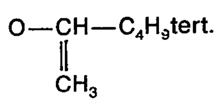 | $-CO_2CH_3$ | $-CH_3$ | 110 |
| 231 | 2′,4′,5′-Trichlorphenoxycarboxyl | $-O-CH_2-C_3H_7-$i | $-CO_2CH_3$ | $-CH_3$ | 70-72 |
| 247 | H | $S-C_4H_9$tert. | $-CO_2CH_3$ | $-CH_3$ | 96-98 |

Die erfindungsgemäßen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, zum Beispiel Benzol, Toluol, Xylol, Paraffin, Tetrahydronaphthalin, alkylierte Naphthaline oder deren Derivate, zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können auch Emulsionskonzentrate, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht :

Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salz von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooktylphenol-, Octylphenol-, Nonylphenol, alkylphenolpolyglykoläther, Tributylphenylpolyglykoläther, Alkalarylpolyätheralkohole, Isotridecylalkohol, Fettalkoholäthylenoxid-Kondensate, äthoxyliertes Rizinusöl, Polyoxyäthylenalkyläther, äthoxyliertes, Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie getreidemehle, Baumrinden-, Holz- und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

### Beispiel 4

Man vermischt 90 Gewichtsteile der Verbindung 5 mit 10 Gewichtsteilen N-Methyl-α-pyrrolidon und erhält eine Lösung, die zur Anwendung in Form kleinster Tropfen geeignet ist.

### Beispiel 5

20 Gewichtsteile der Verbindung 6 werden in einer Mischung gelöst, die aus 80 Gewichtsteilen Xylol, 10 Gewichtsteilen des Anlagerungsproduktes von 8 bis 10 Mol Äthylenoxid an 1 Möl Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

### Beispiel 6

20 Gewichtsteile der Verbindung 7 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in

100 000 Gewichtsteilen Wasser erhält man eine wäßrige Dispersion, die 0,02 Gewichtsprozent des Wirkstoffs enthält.

Beispiel 7

20 Gewichtsteile der Verbindung 5 werden in einer Mischung gelöst, die aus 25 Gewichtsteilen Cyclohexanol, 65 Gewichtsteilen einer Mineralölfraktion vom Siedepunkt 210 bis 280° C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 8

20 Gewichtsteile des Wirkstoffs 6 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen des Mischung in 20 000 Gewichtsteilen Wasser erhält man eine Spritzbrühe, die 0,1 Gewichtsprozent des Wirkstoffs enthält.

Beispiel 9

3 Gewichtsteile der Verbindung 7 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 10

30 Gewichtsteile der Verbindung 5 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 11

Gewichtsteile des Wirkstoffs 6 werden mit 10 Teilen Natriumsalz eines Phenolsulfonsäure-harnstoffformaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 12

20 Teile des Wirkstoffs 7 werden mit 2 Teilen Calciumsalz der Dodecylbenzolsulfonsäure, 8 Teilen Fettalkohol-polyglykoläther, 2 Teilen Natriumsalz eines Phenolsulfonsäureharnstoff-formaldehyd-Kondensats und 68 Teilen eines paraffinischen Mineralöls innig vermischt. Man erhält eine stabile ölige Dispersion.

Die neuen Verbindungen zeigen herbizide Wirkungen und eignen sich zur Beseitigung und Unterdrückung von unerwünschtem Pflanzenwuchs auf Kulturflächen oder unbebautem Land. Dabei ist es selbstverständlich, daß einzelne Wirkstoffe unterschiedliche Wirkungsintensitäten aufweisen oder in ihrer Wirkung gegenüber unerwünschten Pflanzen oder Kulturpflanzen differieren. Ihr Einfluß auf unerwünschte Pflanzen wird in den nachstehenden Tabellen erläutert, welche Ergebnisse aus Gewächshausversuchen darstellen.

Als Kulturgefäße dienten Plastikblumentöpfe mit 300 $cm^3$ Inhalt, die mit lehmigem Sand mit etwa 1,5 % Humus gefüllt wurden. Die Samen der Testpflanzen entsprechend Tabelle 1 wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen auf die Erde gespritzt.

Nach dem Aufbringen der Mittel wurden die Töpfe leicht beregnet, um Keimung und Wachstum der Pflanzen anzuregen und gleichzeitig die Wirkstoffe zu aktivieren. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde, und verhinderte das Verdampfen leicht flüchtiger Wirkstoffe.

Zum Zwecke der Nachauflaufbehandlung zog man die Pflanzen je nach Wuchsform in den Versuchsgefäßen erst bis zu einer Höhe von 3 bis 10 cm an und behandelte sie danach. Eine Abdeckung unterblieb. Die Aufstellung der Versuchstöpfe erfolgt im Gewächshaus, wobei für wärmeliebende Arten wärmere Bereiche des Gewächshauses (25 bis 40° C) und für solche gemäßigter Klimate 15 bis 30° C bevorzugt wurden. Die Versuchsperiode erstreckte sich über 4 bis 6 Wochen. Während dieser Zeit wurden die Pflanzen gepflegt und ihre Reaktion auf die einzelnen Wirkstoffe wurde ausgewertet. Die folgenden Tabellen enthalten die Prüfsubstanzen, die jeweiligen Dosierungen in kg/ha Aktivsubstanz und die Testpflanzenarten. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Sproßteile.

Ergebnis

Die neuen Pyrazol(thio)-ätherderivate entfalten interessante herbizide Eigenschaften bei Vor- und Nachauflaufanwendung. Sie umfassen hierbei sowohl breitblättrige wie grasartige unerwünschte Pflanzen und sparen dabei gewisse Kulturpflanzen selektiv und schonend aus, obwohl diese mit den Wirkstoffen in direkte Berührung kommen. Der Schwerpunkt der Anwendung liegt in der Nachauflaufbehandlung der unerwünschten Pflanzen, gleichgültig ob auf den behandelten Flächen Kulturpflanzen wachsen oder nicht.

Sind gegenüber den Wirkstoffen weniger tolerante Kulturpflanzen vorhanden, so können auch Ausbringungstechniken angewandt werden, bei welchem die Mittel mit Hilfe der Spritzgeräte so gespritzt werden, daß die Blätter empfindlicher Kulturpflanzen nach Möglichkeit nicht getroffen werden, während sie auf die darunterliegende Bodenfläche oder dort wachsende unerwünschte Pflanzen gelangen (post directed, lay-by).

Das breite Spektrum bekämpfbarer Arten aus den verschiedensten botanischen Familien macht die Mittel zudem brauchbar zur Beseitigung unerwünschten Kraut- und Graswuchses in verholzten Baum- und Strauchkulturen sowie Zuckerrohr.

Weiterhin bietet sich die Anwendung auf kulturfreien Flächen wie Industrie- und Gleisanlagen, Park- und Lagerplätzen, Wegen, Grabenrändern und Kahlschlägen an. Es ist hierbei mehr eine Frage der Dosierung, ob der Pflanzenwuchs völlig eliminiert oder lediglich in seinem Wachstum unterdrückt und zurückgehalten wird, ohne die pflanzen abzutöten.

Unter den geprüften Substanzen befinden sich auch solche, welche sich als Austrocknungsmittel für grüne Blätter und Stengel eignen (Desiccants). Solche Mittel dienen beispielsweise zur Abtötung von Kartoffelkraut vor der maschinellen Kartoffelernte, zur Bekämpfung von Unkrautwuchs in reifen Getreidefeldern vor der Ernte, zur Beschleunigung der Abtrocknung von Sojabohnen vor dem Mähdrusch und zur Beseitigung von grünen Pflanzenteilen in pflückreifen Baumwollkulturen vor der Ernte.

In Anbetracht der Vielseitigkeit der Applikationsmethoden können die erfindungsgemäßen Mittel oder diese enthaltende Mischungen außer bei den in den Tabellen aufgeführten Nutzpflanzen noch in einer weiteren großen Zahl von Kulturpflanzen zur Beseitigung unerwünschten Pflanzenwuchses eingesetzt werden. Die Aufwandmengen können dabei von 0,1 bis 15 kg/ha und mehr je nach dem Bekämpfungsobjekt schwanken.

Im einzelnen seien folgende Nutzpflanzen genannt :

| Botanischer Name | Deutscher Name | Englischer Name |
|---|---|---|
| Allium capa | Küchenzwiebel | onions |
| Ananas comosus | Ananas | pineapple |
| Arachis hypogaea | Erdnus | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape |
| Brassica napus var. napobrassica | Kohlrübe | |
| Brassica napus var. rapa | Wieße Rübe | turnips |
| Brassica rapa var. silvestris | Rübsen | |
| Camellia sinensis | Tsestrauch | tea plants |
| Carthamus tinctorius | Saflor - Färberdistel | safflower |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Caffee | coffee plants |

| Botanischer Name | Deutscher Name | Englischer Name |
|---|---|---|
| Cucumis melo | Melone | melons |
| Cucumis sativus | Curxe | cucumber |
| Cynodon dactylon | Bermudagras | Bermudagrass in turfs and lawns |
| Daucus carota | Möhre | carrots |
| Elasis guineensis | Ölpalme | oil palms |
| Fragaria vesca | Erdbeere | strawberries |
| Glycine max | Sojabohne | soybeans |
| Gossypium hirsutum | | |
| (Gossypium arboreum | Baumwolle | cotton |
| Gossypium herbaceum | | |
| Gossypium vitifolium) | | |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Lactuca sativa | Kopfsalat | lettuce |
| Leculinaris | Linse | lentils |
| Linum usitatissimum | Paserlein | flax |
| Lycopersicon lycopersicum | Tomate | tomato |
| Malus spp. | Apfel | apple trees |
| Manibot esculenta | Maniok | cassava |
| Medicago sativa | Luzerne | alfalfa (lucerne) |
| Mentha piperita | Pfefferminze | peppermint |
| Musa spp. | Öbst- und Mehlbanane | banana plants |
| Oicotiana tabacum | Tabak | tobacco |
| (N. rustica) | | |
| Olea europaea | Olbaum | olive trees |
| Oryza sativa | Reis | rice |
| Panicum miliaceum | Rispenhirse | |
| Phaseolus lunatus | Mondbohne | limabeans |
| Phaseolus mungo | Urübohne | mungbeans |
| Phaseolus vulgaris | Buschbohnen | snapbeans, green beans, dry beans |
| Pennisetum glaucum | Perl- oder Rohrkolbenhirse | |
| Petroselinum crispum | Wurzelpetersilie | parsley |
| spp. tuberosum | | |
| Picea abies | Rotfichte | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesame | Sesame |
| Solanum tuberosum | Kartoffel | Irish potatoes |
| Sorghum bicolor (s. vulgare) | Monrenhirse | sorghum |
| Sorghum dochna | Zuckerhirse | |
| Spinacia oleracea | Spinat | spinach |
| Theotroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (v. unguiculata) | Kuhbohne | cow peas |
| Vitis vinifera | Weinrebe | grapes |
| Zea mays | Mais | Indian corn, sweet corn, maize |

Zur weiteren Verbreiterung des Wirkungsspektrums der neuen Wirkstoffe, zur Erzielung synergistischer Effekte oder zum Verbessern der Dauerwirkung im Boden, lassen sich zahlreiche andere Herbizide oder wachstumsregulierende Verbindungen als Mischungs- und Kombinationspartner heranziehen. Je nach Einsatzgebiet und Bekämpfungsvorhaben bieten sich nachstehende Substanzen oder ähnliche Derivate als Mischungspartner an :

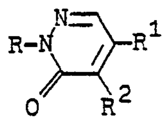

| R | $R^1$ | $R^2$ |
|---|---|---|
| 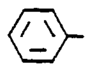 | $NH_2$ | Cl |
| 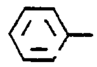 | $NH_2$ | Br |
| 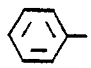 | $OCH_3$ | $OCH_3$ |
| 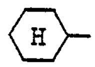 | $OCH_3$ | $OCH_3$ |
| 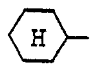 | $NH_2$ | Cl |
| 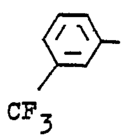 | $NHCH_3$ | Cl |
| 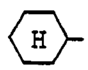 | $NH_2$ | Br |
| 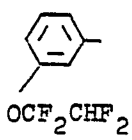 | $NH.CH_3$ | Cl |

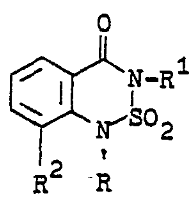

| R | $R^1$ | $R^2$ | |
|---|---|---|---|
| H | 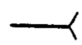 | H | (Salze) |
| H | 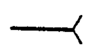 | $CH_3$ | " |
| H | 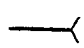 | Cl | " |
| H | 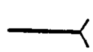 | F | " |
| $CH_2OCH_3$ | 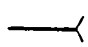 | F | |
| $CH_2OCH_3$ | 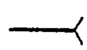 | H | |
| CN | 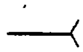 | H | |
| CN | 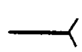 | Cl | |

| | | |
|---|---|---|
| CN | 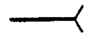 | F |
| $CH_2N_3$ | 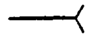 | H |

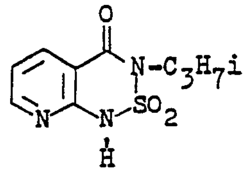

(Salze)

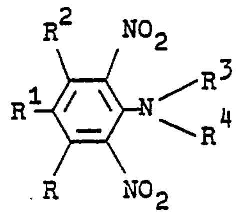

| R | $R^1$ | $R^2$ | $R^3$ | $R^4$ |
|---|---|---|---|---|
| H | $F_3C$ | H | $C_2H_5$ | $C_4H_9$ |
| H | $F_3C$ | H | $n.C_3H_7$ | $n.C_3H_7$ |
| H | $F_3C$ | H | $-CH_2-CH_2Cl$ | $n.C_3H_7$ |
| H | $SO_2NH_2$ | H | $n.C_3H_7$ | $n.C_3H_7$ |
| H | $F_3C$ | H | $nC_3H_7$ | 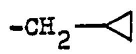 |
| $H_3C$ | $H_3C$ | H | H | 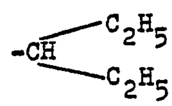 |

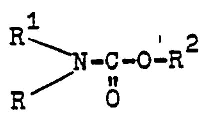

| R | $R^1$ | $R^2$ |
|---|---|---|
| 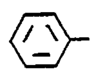 | H | $iC_3H_7$ |
| $CH_3$ | H | 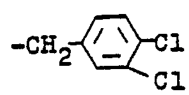 |
| 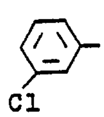 | H | 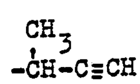 |
| 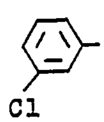 | H | $iC_3H_7$ |
| 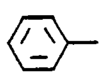 | H | 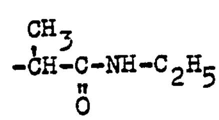 |

| R | $R^1$ | $R^2$ |
|---|---|---|
| 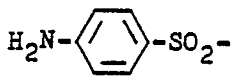 | H | $CH_3$ |

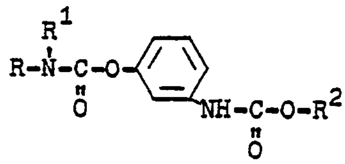

| | | |
|---|---|---|
| 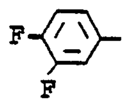 | H | $C_2H_5$ |
| 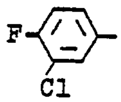 | H | $C_2H_5$ |
| 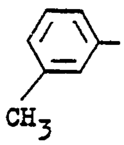 | H | $CH_3$ |
| 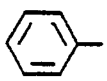 | H | $C_2H_5$ |
| 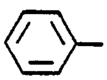 | $CH_3$ | $CH_3$ |
| 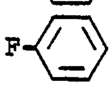 | H | $CH_3$ |

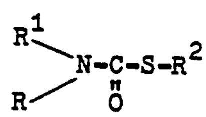

| R | $R^1$ | $R^2$ |
|---|---|---|
| $iC_3H_7$ | $iC_3H_7$ | $CH_2-CCl=CCl_2$ |
| $iC_3H_7$ | $iC_3H_7$ | $CH_2-CCl=CHCl$ |
| $n.C_3H_7$ | $n.C_3H_7$ | $C_2H_5$ |

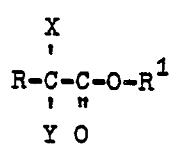

| R | X | Y | $R^1$ |
|---|---|---|---|
| $CH_3$ | Cl | Cl | Na |
| Cl | Cl | Cl | Na |
| 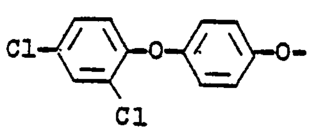 | H | $CH_3$ | $CH_3$ |

| | | | |
|---|---|---|---|
| 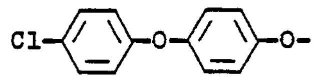 | H | $CH_3$ | 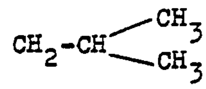 |
| 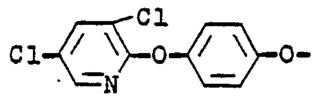 | H | $CH_3$ | Na |
| 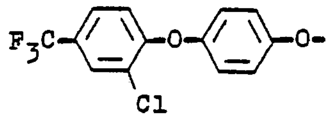 | H | $CH_3$ | Na |
| 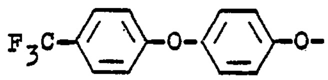 | H | $CH_3$ | $CH_3$ |

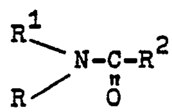

| R | $R^1$ | $R^2$ |
|---|---|---|
| 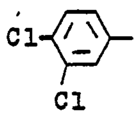 | H | 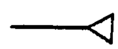 |

| R | $R^1$ | $R^2$ |
|---|---|---|
| 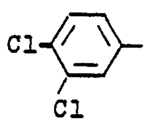 | H | $C_2H_5$ |
| 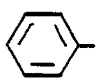 | $CH_3$<br>$-CH-C=CH$ | $CH_2Cl$ |
| 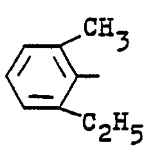 | $CH_3$<br>$-CH-CH_2-OCH_3$ | $CH_2Cl$ |
| 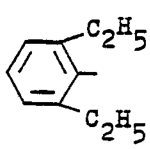 | $-CH_2OCH_3$ | $CH_2Cl$ |
| 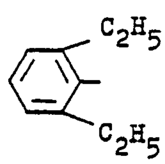 | $-CH_2-C-OC_2H_5$<br>O | $CH_2Cl$ |
| 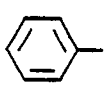 | $iC_3H_7$ | $CH_2Cl$ |

| | | |
|---|---|---|
| 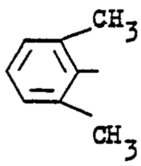 | $-CH_2-CH_2-OCH_3$ | $Cl_2Cl$ |
| $C_2H_5$ | $C_2H_5$ | 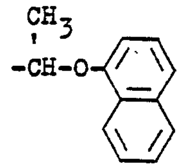 |

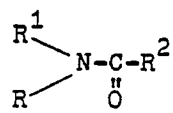

| R | $R^1$ | $R^2$ |
|---|---|---|
| 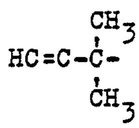 | H | 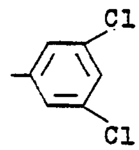 |
| 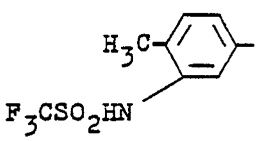 | H | $CH_3$ |
| 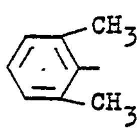 | 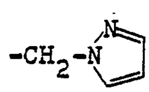 | $CH_2Cl$ |
| 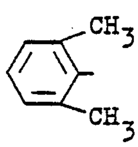 | 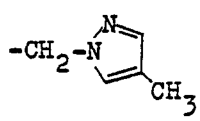 | $CH_2Cl$ |
| 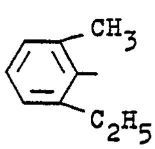 | 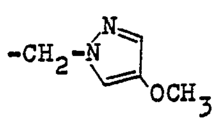 | $CH_2Cl$ |
| 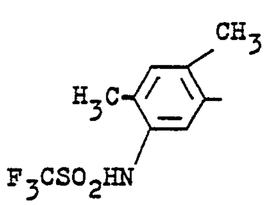 | H | $CH_3$ |

| R | $R^1$ | $R^2$ |
|---|---|---|
| 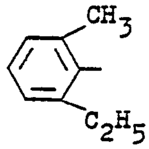 | 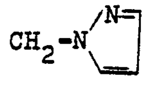 | $CH_2Cl$ |

| | | |
|---|---|---|
| 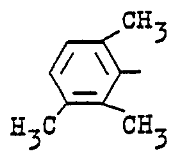 | 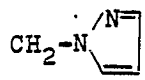 | $CH_2Cl$ |
| 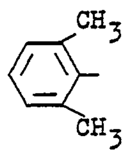 | 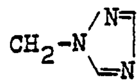 | $CH_2Cl$ |

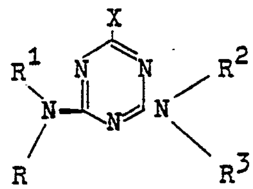

| R | $R^1$ | X | $R^2$ | $R^3$ |
|---|---|---|---|---|
| H | $C_2H_5$ | $SCH_3$ | H | $C_2H_5$ |
| H | $iC_3H_7$ | $SCH_3$ | H | $C_2H_5$ |
| H | $iC_3H_7$ | Cl | H | $C_2H_5$ |
| H | $iC_3H_7$ | Cl | H | 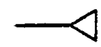 |
| H | $C_2H_5$ | Cl | H | $C_2H_5$ |
| H | $C_2H_5$ | Cl | H | 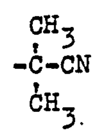 |
| H | $iC_3H_7$ | $OCH_3$ | H | $iC_3H_7$ |

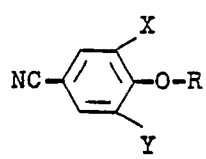

| X | Y | R | |
|---|---|---|---|
| Br | Br | H | (Salze) |
| I | I | H | " |
| Br | Br | 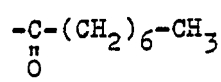 | |

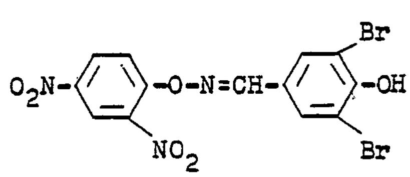 Salze, Ester

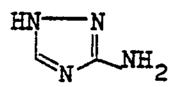

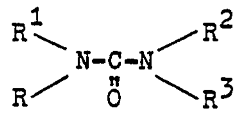

| R | $R^1$ | $R^2$ | $R^3$ |
|---|---|---|---|
| 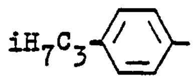 | H | $CH_3$ | $CH_3$ |
| 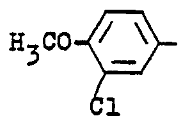 | H | $CH_3$ | $CH_3$ |
| 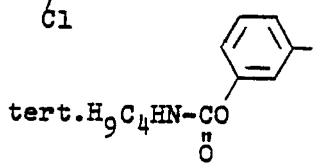 | H | $CH_3$ | $CH_3$ |

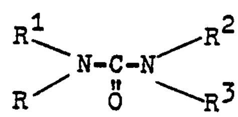

| R | $R^1$ | $R^2$ | $R^3$ |
|---|---|---|---|
| 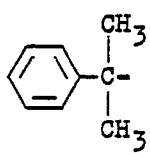 | H | H | 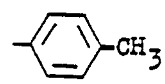 |
| 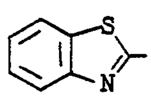 | $CH_3$ | $CH_3$ | H |
| 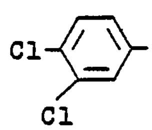 | H | $CH_3$ | $CH_3$ |
| 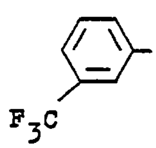 | H | $CH_3$ | $CH_3$ |
| 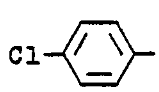 | H | $CH_3$ | 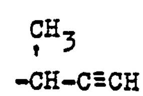 |
| 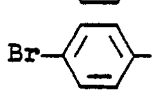 | H | $CH_3$ | $OCH_3$ |
| 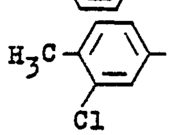 | H | $CH_3$ | $CH_3$ |

| | | | |
|---|---|---|---|
| 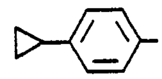 | H | $CH_3$ | $CH_3$ |
| 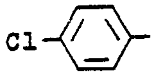 | H | $CH_3$ | $OCH_3$ |
| 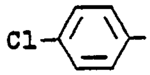 | H | $CH_3$ | $CH_3$ |

| R | $R^1$ | $R^2$ | $R^3$ |
|---|---|---|---|
| 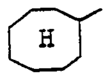 | H | $CH_3$ | $CH_3$ |
| 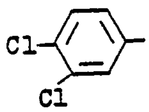 | H | $CH_3$ | $OCH_3$ |

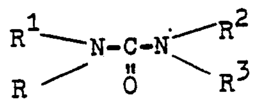

| R | $R^1$ | $R^2$ | $R^3$ |
|---|---|---|---|
| 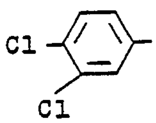 | H | $CH_3$ | H |
| 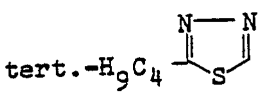 | $CH_3$ | $CH_3$ | H |
| 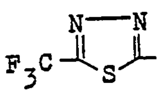 | $CH_3$ | $CH_3$ | H |
| 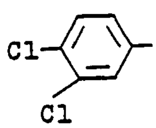 | H | $C_2H_5$ | $C_2H_5$ |
| 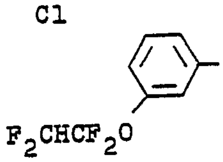 | H | $CH_3$ | $CH_3$ |

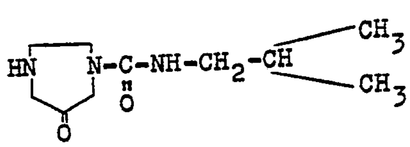

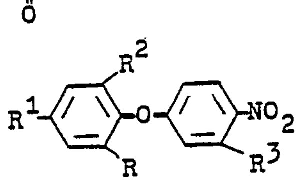

| R | $R^1$ | $R^2$ | $R^3$ |
|---|---|---|---|
| Cl | $CF_3$ | H | COOH Salze |
| Cl | Cl | H | H |

| | | | |
|---|---|---|---|
| Cl | Cl | H | 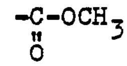 |
| H | $CF_3$ | Cl | $OC_2H_5$ |

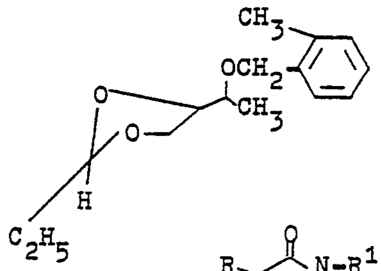

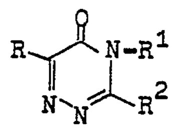

| R | $R^1$ | $R^2$ |
|---|---|---|
| tert.-$C_4H_9$ | $NH_2$ | $SCH_3$ |
| 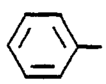 | $NH_2$ | $CH_3$ |
| 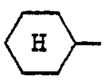 | $NH_2$ | $SCH_3$ |

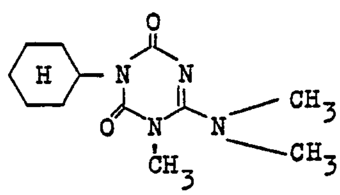

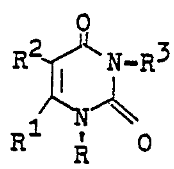

| R | $R^1$ | $R^2$ | $R^3$ |
|---|---|---|---|
| H | $CH_3$ | Br | 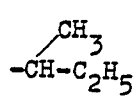 |
| H | $CH_3$ | Br | i$C_3H_7$ |
| H | $CH_3$ | Cl | tert.-$C_4H_9$ |
| H | $CH_3$ | Cl | 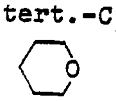 |

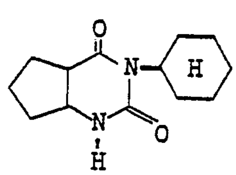

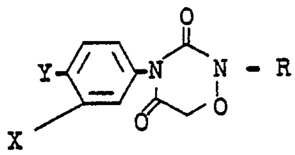

| X | Y | R |
|---|---|---|
| $CF_3$ | H | $CH_3$ |
| H | F | $CH_3$ |

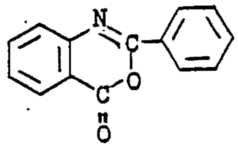

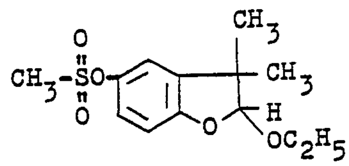

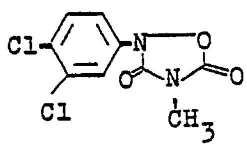

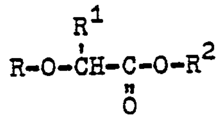

| R | $R^1$ | $R^2$ |
|---|---|---|
| 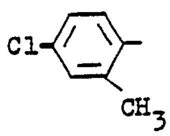 | $CH_3$ | H Salze, Ester, Amide |
| 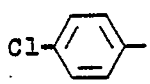 | H | H " |

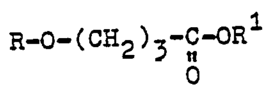

| R | $R^1$ | |
|---|---|---|
| 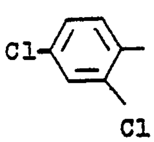 | H | Salze, Ester, Amide |
| 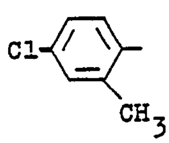 | H | " |

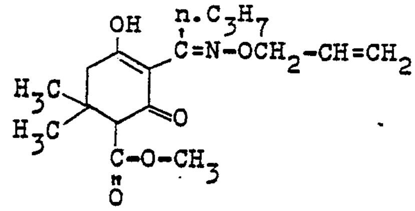

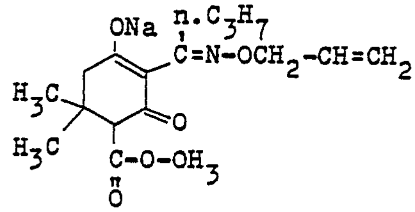

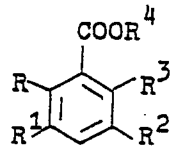

| R | $R^1$ | $R^2$ | $R^3$ | $R^4$ |
|---|---|---|---|---|
| H | Cl | $NH_2$ | Cl | H Salze, Ester, Amide |
| Cl | Cl | H | Cl | Na |
| Cl | H | Cl | $OCH_3$ | H Salze, Ester, Amide |
| Cl | Cl | H | Cl | $H.(CH_3)_2NH$ |

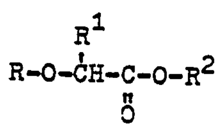

| R | $R^1$ | $R^2$ | |
|---|---|---|---|
| 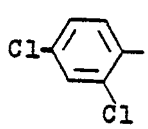 | $CH_3$ | H | Salze, Ester, Amide |
| 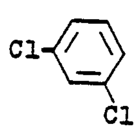 | H | H | " |
| 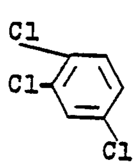 | H | H | " |

| | | | |
|---|---|---|---|
| 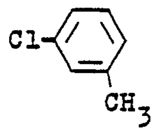 | H | H | " |
| 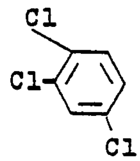 | $CH_3$ | H | " |

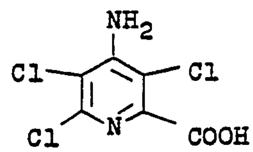

Salze, Ester

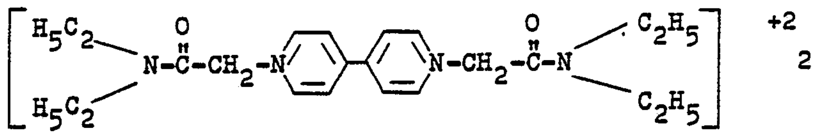

2 $Cl^-$

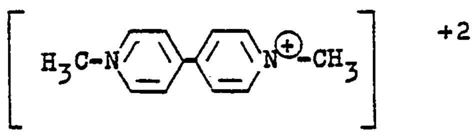

2 $CH_3OSO_3^-$

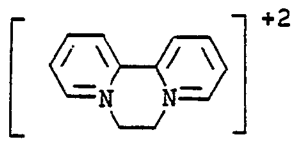

2 $Br^-$

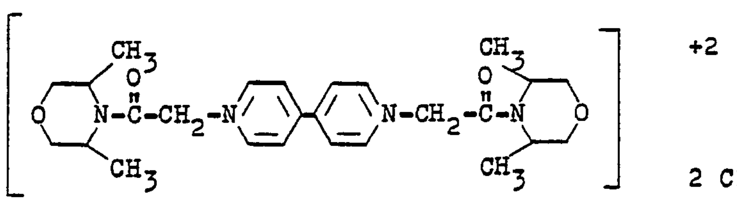

2 $Cl^-$

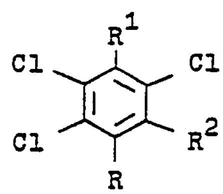

| R | $R^1$ | $R^2$ |
|---|---|---|
| $COOCH_3$ | $COOCH_3$ | Cl |

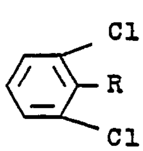

R = CN

R = $CSNH_2$

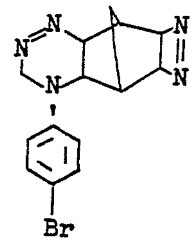

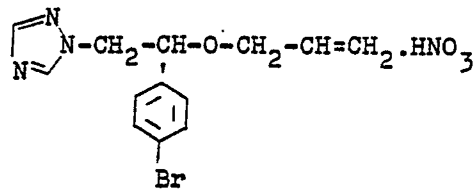

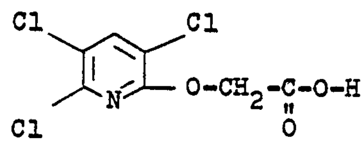

Salze, Ester, Amide

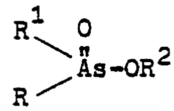

| R | $R^1$ | $R^2$ |
|---|---|---|
| OH | $CH_3$ | Na |
| $CH_3$ | $CH_3$ | Na |
| $CH_3$ | $CH_3$ | OH |
| ONa | $CH_3$ | Na |

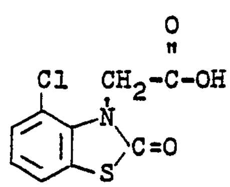

Salze

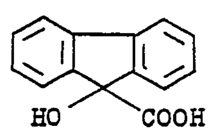

(Salze, Ester)

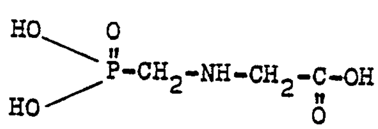

(Salze)

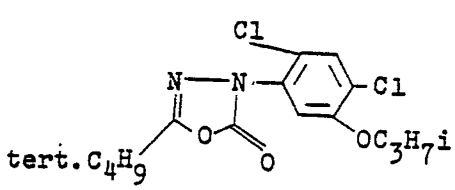

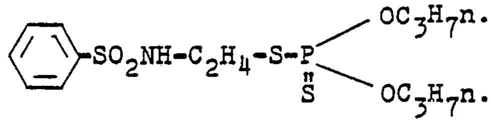

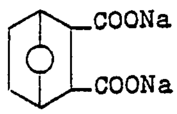

und andere Salze

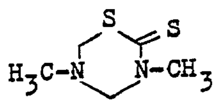

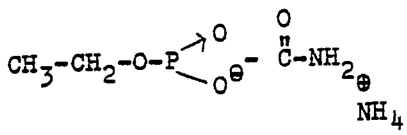

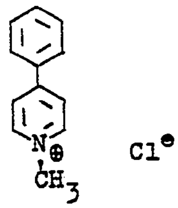

$NH_4SCN$

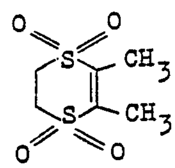

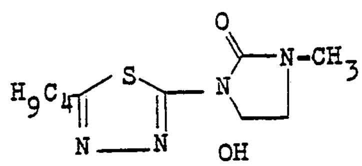

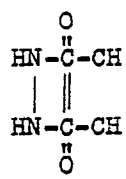

(Salze)

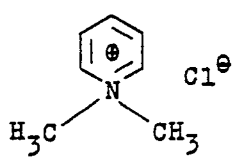

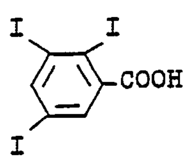

Salze, Ester, Amide

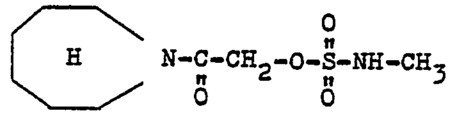

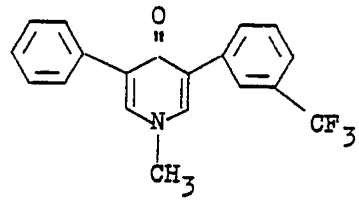

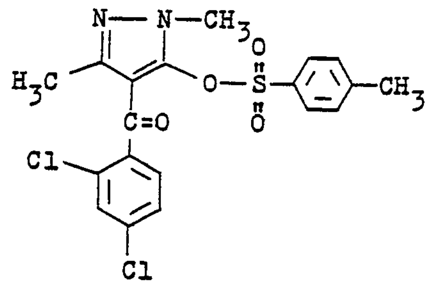

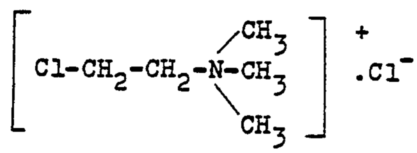

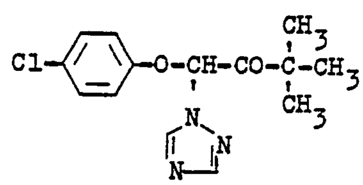

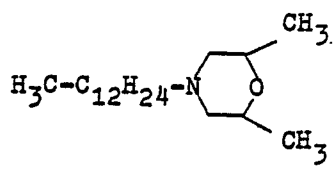

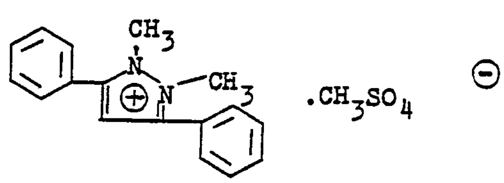

Außerdem ist es nützlich, die neuen Wirkstoffe allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralstofflösungen, welche zur Behebung von Ernährungs- oder Spurenelementmängeln eingesetzt werden.

Außerdem ist es unter gewissen Umständen vorteilhaft, die Mittel einzeln oder in den genannten Kombinationen gemeinsam mit festen oder flüssigen handelsüblichen Mineraldüngern gemischt auszubringen.

TABELLE 1
Liste der Pflanzennamen

| Botanischer Name | Abkürz. in Tab. | Deutscher Name | Englischer Name |
|---|---|---|---|
| Abutilon theophrasti | Abut. theo. | chinesischer Hanf | velvet leaf |
| Amaranthus retroflexus | Amar. ret. | zurückgekrümmter Fuchsschwanz | redroot pigweed |
| Arachys hypogaea | Arachys hyp. | Erdnuß | peanuts (groundnuts) |
| Avena fatua | Avena fatua | Flughafer | wild oats |
| Centaurea cyanus | Centaurea cyanus | Kornblume | cornflower |
| Chenopodium album | Chenopodium album | weißer Gänsefuß | lambsquarters |
| Chrysanthemum segetum | Chrys. seg. | Saatwucherblume | corn marigold |
| Echinochloa crus galli | Echin. c. g. | Hühnerhirse | barnyardgrass |
| Eleusine indica | Eleus. ind. | | goosegrass |
| Euphorbia geniculata | Euph. genic. | südamerikanische Wolfsmilchart | Southamerican member of the spurge family |
| Ipomoea spp. | Ipomoea spp. | Prunkwindearten | morningglory |
| Lolium multiflorum | Lolium mult. | italienisches Raygrass | annual raygrass |
| Sesbania exaltata | Sesbania exaltata | Turibaum | hemp sesbania(coffeeweed) |
| Sinapis alba | Sinapis alba | weißer Senf | white mustard |
| Sorghum bicolor | Sorghum bicolor | Mohrenhirse (Kulturhirse) | sorghum |
| Stellaria media | Stellaria media | Vogelsternmiere | chickweed |
| Triticum aestivum | Triticum aestivum | Weizen | wheat |
| Zea mays | Mais | Mais | Indian corn |
| Lamium amplexicaule | Lamium amplex. | stengelumfassende Taubnessel | henbit |
| Lamium spp. | Lamium spp. | Taubnesselarten | dead nettle |
| Solanum nigrum | Solanum nigrum | schwarzer Nachtschatten | black nightshade |
| Gossypium hirsutum | Gossypium hirsutum | Baumwolle | cotton |

TABELLE 2
Selektive Bekämpfung von Avena fatua und anderen Unkräutern in Weizen bei Nachauflaufanwendung im Gewächshaus

| Wirkstoff-Nr. | kg/ha | Testpflanzen und % Schädigung: Triticum aestivum | Avena fatua | Chenopodium album | Stellaria media |
|---|---|---|---|---|---|
| 90 | 0,5 | 0 | 80 | 98 | 95 |
| | 1,0 | - | 100 | 98 | 100 |
| | 2,0 | 0 | 100 | - | 100 |
| 49 | 0,5 | 0 | 80 | 98 | 100 |
| | 1,0 | - | 85 | 98 | 100 |
| | 2,0 | 0 | 100 | - | 100 |
| 51 | 0,5 | 0 | 50 | 98 | 100 |
| | 1,0 | - | 95 | 98 | 100 |
| | 2,0 | 0 | 95 | - | 100 |
| 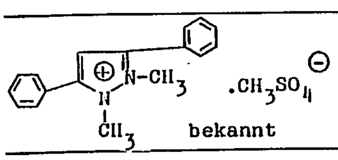 bekannt | 0,5 | 6 | 33 | - | 18 |
| | 1,0 | 16 | 56 | - | 20 |
| | 2,0 | 28 | 72 | - | 35 |

0 = keine Schädigung, 100 = Pflanzen abgestorben

TABELLE 3

Selektive Bekämpfung von unerwünschten Pflanzen in Erdnüssen bei Nachauflaufanwendung im Gewächshaus

| Wirkstoff-Nr. | kg/ ha | Testpflanzen und % Schädigung Arachys hyp. | Amar. ret. | Abut. theo. | Chrys. seg. | Echin. c.g. | Eleu. ind. | Euph. genic. | Ipomoea spp. | Sesbania exaltata |
|---|---|---|---|---|---|---|---|---|---|---|
| 40 | 0,5 | 5 | 100 | 100 | 100 | 100 | 98 | 100 | 92 | 100 |
| | 1,0 | 5 | 100 | 100 | 100 | 100 | 100 | 100 | 92 | 100 |
| | 2,0 | 10 | 100 | - | 100 | 100 | - | 100 | 100 | 100 |
| 76 | 0,5 | 0 | 100 | 100 | 100 | 100 | 100 | 98 | 75 | 100 |
| | 1,0 | 2 | 100 | 100 | 100 | 100 | 100 | 100 | 75 | 100 |
| | 2,0 | 10 | 100 | - | 100 | 100 | - | 100 | 100 | 100 |
| 44 | 0,5 | 5 | 50 | 100 | 100 | 90 | 100 | 100 | 90 | 100 |
| | 1,0 | 5 | 100 | 100 | 100 | 100 | 100 | 100 | 92 | 100 |
| | 2,0 | 10 | 100 | - | 100 | 100 | - | 100 | 100 | 100 |
| 12 | 0,5 | 5 | 100 | 100 | 100 | 99 | 100 | 100 | 85 | 100 |
| | 1,0 | 5 | 100 | 100 | 100 | 100 | 100 | 100 | 100 | 100 |
| | 2,0 | - | 100 | - | 100 | 100 | - | 100 | 100 | 100 |
| 53 | 0,5 | 0 | 100 | 98 | - | 100 | - | - | 100 | 100 |
| | 1,0 | 0 | 100 | 100 | - | 100 | - | - | 100 | 100 |
| | 2,0 | - | - | - | - | 100 | - | - | 100 | - |
| 50 | 0,5 | 0 | 100 | 65 | 100 | 99 | 85 | 98 | 92 | 100 |
| | 1,0 | 0 | 100 | 100 | 100 | 100 | 100 | 100 | 100 | 100 |
| | 2,0 | 10 | 100 | - | 100 | 100 | - | 100 | - | 100 |
| 10 | 0,5 | 0 | 100 | 100 | 100 | 100 | 98 | 100 | 100 | 100 |
| | 1,0 | 5 | 100 | 100 | 100 | 100 | 100 | 100 | 100 | 100 |
| | 2,0 | 0 | 100 | - | 100 | 100 | - | 100 | 100 | 100 |
| 63 | 0,5 | 5 | 100 | 100 | 100 | 100 | 100 | 100 | 98 | 100 |
| | 1,0 | 5 | 100 | 100 | 100 | 100 | 100 | 100 | 98 | 100 |
| | 2,0 | 10 | 100 | - | 100 | 100 | - | 100 | 100 | 100 |
| 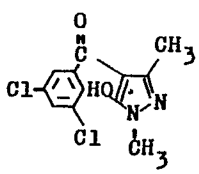 | 1,0 | 0 | 10 | - | - | 20 | - | 20 | 0 | 0 |
| | 2.0 | 0 | 20 | - | - | - | - | 20 | 0 | 0 |
| bekannt (DE-OS 2 513 750) | | | | | | | | | | |
| 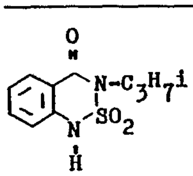 | 1,0 | 0 | 70 | 100 | 100 | 0 | - | 33 | 43 | 80 |
| | 2,0 | 0 | 100 | 100 | 100 | 0 | - | 37 | 55 | 98 |
| bekannt (E-PS 1 542 836) | | | | | | | | | | |

0 = keine Schädigung, 100 = Pflanzen abgestorben

TABELLE 4

Beseitigung von unerwünschten Pflanzen in Kultursorghum und Mais bei Nachauflaufanwendung

| Wirkstoff-Nr. | kg/ha | Testpflanzen und % Schädigung | | | | | |
|---|---|---|---|---|---|---|---|
| | | Sorghum bicolor | Zea mays | Centaurea cyanus | Chenopodium album | Sesbania exaltata | Sinapis alba |
| 7 | 0,25 | 0 | 12 | 95 | 95 | 97 | - |
| | 0,5 | 10 | 17 | 98 | 100 | 97 | 100 |
| | 1,0 | - | - | 98 | 100 | 100 | 100 |
| 44 | 0,5 | 15 | 15 | 98 | 100 | 100 | 100 |
| | 1,0 | 20 | 18 | 98 | 100 | 100 | 100 |
| 52 | 0,5 | 0 | 10 | 98 | 98 | 80 | 98 |
| | 1,0 | 10 | 10 | 98 | 98 | 100 | 100 |
| 49 | 0,5 | 0 | 20 | 75 | 98 | 90 | 95 |
| | 1,0 | 0 | 20 | 80 | 98 | 100 | 95 |
| 51 | 0,5 | 10 | 15 | 65 | 98 | 80 | 99 |
| | 1,0 | 15 | 20 | 100 | 98 | 100 | 99 |
| 78 | 0,5 | 0 | 20 | 100 | 98 | 100 | 99 |
| | 1,0 | 0 | 20 | 100 | 98 | 100 | 99 |
| 79 | 0,5 | 15 | 25 | 98 | 98 | 100 | 99 |
| | 1,0 | 15 | 25 | 98 | 98 | 100 | 99 |
| 90 | 0,5 | 0 | 10 | 60 | 98 | - | - |
| | 1,0 | 0 | 10 | 75 | 98 | 100 | 80 |

0 = keine Schädigung, 100 = Pflanzen abgestorben

TABELLE 5

Anwendung im Vorauflaufverfahren im Gewächshaus

| Wirkstoff-Nr. | kg /ha | Testpflanzen und % Schädigung | | |
|---|---|---|---|---|
| | | Echin. c.g. | Lolium mult. | Sinapis alba |
| 15 | 3,0 | 100 | - | 100 |
| 44 | 3,0 | 70 | 100 | 100 |
| 9 | 3,0 | 70 | 100 | 100 |
| 12 | 3,0 | - | 100 | 100 |
| 50 | 3,0 | 70 | 100 | 100 |
| 53 | 3,0 | 100 | 100 | 100 |
| 56 | 3,0 | 70 | - | 100 |
| 52 | 3,0 | 100 | - | 100 |
| 10 | 3,0 | 100 | 100 | 100 |
| 63 | 3,0 | - | 100 | 100 |

Fortsetzung von Tabelle 5

| Wirkstoff-Nr. | kg/ha | Testpflanzen und % Schädigung Echin. c.g. | Lolium mult. | Sinapis alba |
|---|---|---|---|---|
| 7 | 3,0 | - | 100 | 100 |
| 75 | 3,0 | 100 | 100 | 100 |
| 76 | 3,0 | 100 | - | 100 |
| 78 | 3,0 | 90 | - | 90 |
| 79 | 3,0 | 100 | 80 | 90 |
| 40 | 3,0 | 100 | 100 | 100 |
| 49 | 3,0 | 100 | - | 100 |

0 = Pflanzen ohne Wirkung, 100 = Samen nicht gekeimt bzw. Pflanzen vollkommen abgestorben

TABELLE 6

Bekämpfung unerwünschter Pflanzen bei Nachauflaufanwendung im Gewächshaus

| Wirkstoff-Nr. | kg/ha | Testpflanzen und % Schädigung Echin. c.g. | Ipomoea spp. | Lamium amplex. | Sinapis alba | Stellaria media |
|---|---|---|---|---|---|---|
| 66 | 1,0 | 95 | 95 | 95 | 80 | 80 |
| 69 | 1,0 | 95 | 95 | 95 | 80 | 80 |
| 67 | 1,0 | 90 | 95 | 98 | - | 80 |
| 71 | 1,0 | 95 | 95 | 80 | 70 | 70 |
| 70 | 1,0 | 90 | 95 | 95 | 80 | - |
| 124 | 1,0 | 80 | 80 | 95 | 80 | 80 |
| 123 | 1,0 | 80 | 90 | 95 | 70 | 90 |
| 74 | 1,0 | 90 | 95 | 80 | - | 70 |
| 121 | 1,0 | 90 | 90 | 98 | 80 | 70 |
| 87 | 1,0 | 70 | - | 100 | 98 | 90 |
| 83 | 1,0 | 75 | 95 | 100 | 90 | 100 |
| 54 | 1,0 | 100 | 100 | 100 | 70 | 100 |
| 89 | 1,0 | 90 | 98 | 100 | 98 | 100 |

0 = keine Schädigung, 100 = Pflanzen völlig abgestorben

TABELLE 7

Bekämpfung von Flughafer und anderen unerwünschten Pflanzen in Gramineen-Kulturen bei Nachauflaufanwendung im Gewächshaus

| Wirkstoff-Nr. | kg /ha | Testpflanzen und % Schädigung | | | | | | | |
|---|---|---|---|---|---|---|---|---|---|
| | | Sorgh. bic. | Tritic. aest. | Avena fatua | Chenop. album | Lamium spp. | Sinap. alba | Sesb. exal. | Solan. nig. |
| 182 | 1,0 | 20 | 15 | 88 | - | 85 | 90 | 100 | 100 |
| 179 | 2,0 | 15 | 10 | 80 | 100 | 100 | 95 | 100 | 100 |
| 196 | 1,0 | - | 0 | 100 | 100 | 100 | 98 | 100 | 100 |
| 55 | 1,0 | - | 10 | 95 | 98 | - | 65 | 70 | - |
| 169 | 1,0 | 10 | - | 90 | - | - | 98 | 100 | 100 |
| 23 | 1,0 | 0 | - | 98 | 100 | 100 | 70 | 100 | 95 |
| 42 | 1,0 | 0 | 0 | 90 | 100 | 100 | 65 | 90 | 100 |
| 103 | 1,0 | 0 | 0 | 65 | 98 | 100 | 90 | 100 | 100 |

TABELLE 8

Selektive herbizide Wirkung in Baumwolle bei Nachauflaufanwendung im Gewächshaus

| Wirkstoff-Nr. | kg/ ha | Testpflanzen und % Schädigung | | | | | |
|---|---|---|---|---|---|---|---|
| | | Gossyp. hirs. | Amaranth. retrof. | Echin. c.g. | Ipomoea spp. | Sesb. exal. | Solan. nig. |
| 137 | 1,0 | 0 | - | 100 | 92 | 100 | 100 |
| 191 | 1,0 | 0 | 100 | - | - | 100 | 100 |
| 125 | 2,0 | 0 | 100 | 96 | - | 100 | 100 |
| 122 | 2,0 | 0 | 95 | 100 | 95 | 100 | - |
| 190 | 2,0 | 0 | 100 | 95 | 98 | 100 | 100 |
| 197 | 2,0 | 10 | 50 | 99 | 92 | 100 | - |
| 189 | 2,0 | 10 | - | 95 | 98 | 100 | 100 |
| 68 | 2,0 | 0 | - | 95 | 100 | 100 | 100 |

TABELLE 9

Herbizide Wirkung bei Nachauflaufanwendung im Gewächshaus

| Wirkstoff-Nr. | kg /ha | Testpflanzen und % Schädigung | | | |
|---|---|---|---|---|---|
| | | Centaurea cyanus | Echin. c.g. | Ipomoea spp. | Lolium multifl. |
| 216 | 3,0 | 100 | 100 | 100 | 100 |
| 221 | 3,0 | 100 | 90 | 90 | 100 |

Fortsetzung von Tabelle 9

| Wirkstoff-Nr. | kg/ ha | Testpflanzen und % Schädigung Centaurea cyanus | Echin. c.g. | Ipomoea spp. | Lolium multifl. |
|---|---|---|---|---|---|
| 223 | 3,0 | 90 | 80 | 80 | - |
| 106 | 3,0 | 100 | 80 | 100 | 80 |
| 84 | 3,0 | 100 | 80 | - | 80 |
| 91 | 3,0 | 100 | 70 | 100 | 100 |
| 128 | 3,0 | 100 | 90 | 100 | 90 |
| 170 | 3,0 | 100 | 90 | 100 | 90 |
| 177 | 3,0 | 100 | - | - | 100 |
| 181 | 3,0 | 100 | 100 | 100 | 90 |
| 201 | 3,0 | 100 | 95 | 100 | 100 |
| 211 | 3,0 | 100 | 95 | 100 | 90 |
| 218 | 3,0 | 100 | 100 | 100 | 90 |
| 217 | 3,0 | 100 | 90 | 100 | - |
| 193 | 3,0 | 100 | 100 | 100 | - |
| 192 | 3,0 | 100 | 80 | 100 | 90 |
| 219 | 3,0 | 100 | 90 | 100 | 90 |
| 45 | 3,0 | 100 | 90 | 100 | 100 |
| 195 | 3,0 | 100 | - | 100 | 100 |
| 132 | 3,0 | 100 | 95 | 90 | 60 |
| 212 | 3,0 | 100 | 80 | 95 | 90 |
| 214 | 3,0 | 100 | 95 | 100 | 90 |
| 228 | 3,0 | 100 | 100 | 90 | 90 |
| 231 | 3,0 | 100 | 100 | 90 | 95 |

**Ansprüche für die Vertragsstaaten :** BE, CH, DE, FR, GB, JT, LU, NL, SE.

1. Pyrazolätherderivat der Formel

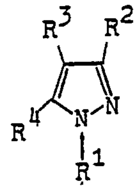

(1)

in welcher $R^1$ Wasserstoff oder $-\underset{\underset{O}{\|}}{C}-R^5$ bedeutet, wobei

$R^5$ Wasserstoff, Alkoxyalkyl, Alkyl oder Halogenalkyl bedeutet,

$R^1$ ferner $-\underset{\underset{O}{\|}}{C}-O-R^6$ bedeutet, wobei

$R^6$ Alkyl oder Phenyl bedeutet, das gegebenenfalls ein- oder mehrfach durch Halogen substituiert ist,

$R^2$ die Gruppe Y-$R^7$ bedeutet, wobei Y Sauerstoff oder Schwefel bedeutet und $R^7$ einen aliphatischen, cycloaliphatischen oder araliphatischen Kohlenwasserstoffrest, Cyclopentylmethyl oder einen Arylrest bedeutet, wobei dieser Arylrest gegebenenfalls ein- oder mehrfach durch Alkyl, Halogen, Halogenalkyl, Alkoxy substituiert ist,

$R^3$ den Methoxycarbonylrest bedeutet und

$R^4$ Wasserstoff oder Methyl bedeutet und die Salze der Pyrazolätherderivate.

2. Herbizid, enthaltend ein Pyrazolätherderivat gemäß Anspruch 1.

3. Herbizid, enthaltend einen festen oder flüssigen Trägerstoff und ein Pyrazolätherderivat gemäß Anspruch 1.

4. Verfahren zur Bekämpfung unerwünschten Pflanzenwuchses, *dadurch gekennzeichnet,* daß man den Boden oder die Pflanzen behandelt mit einem Pyrazolätherderivat gemäß Anspruch 1.

5. Verfahren zur Herstellung eines Pyrazolätherderivates gemäß Anspruch 1, *dadurch gekennzeichnet,* daß man ein Thiokohlensäureesterhydrazid der Formel $R^2-\underset{\underset{S}{\|}}{C}-\underset{\underset{R^1}{|}}{N}-NH_2$ umsetzt mit einer 2-Chlor-1,3-dicarbonylverbindung der Formel $R^4-CO-CHCl-R^3$, wobei $R^1-R^4$ die im Anspruch 1 genannten Bedeutungen haben, und das so erhaltene Pyrazolderivat gegebenenfalls acyliert.

6. Pyrazolätherderivat gemäß Anspruch 1, ausgewählt aus der Gruppe, bestehend aus den Verbindungen 3-(2-Methylpropyloxy)-4-methoxycarbonyl-5-methylpyrazol, 3-Phenyloxy-4-methoxycarbonyl-5-methylpyrazol, 3-(2,2-Dimethylpropyloxy)-4-methoxycarbonyl-5-methylpyrazol, 3-(3-Methoxyphenyloxy)-4-methoxycarbonyl-5-methylpyrazol, 3-(Cyclopentylmethyloxy)-4-methoxycarbonyl-5-methylpyrazol, 1-Acetyl-3-cyclopentylmethyloxy-4-methoxycarbonyl-5-methylpyrazol, 3-(2-Methylcyclohexyloxy)-4-methoxycarbonyl-5-methylpyrazol.

7. Herbizid gemäß Anspruch 2, enthaltend ein Pyrazolätherderivat, ausgewählt aus der Gruppe, bestehend aus den Verbindungen 3-(2-Methyl-propyloxy)-4-methoxycarbonyl-5-methylpyrazol, 3-Phenyloxy-4-methoxy-carbonyl-5-methylpyrazol, 3-(2,2-Dimethylpropyloxy)-4-methoxycarbonyl-5-methylpyrazol, 3-(3-Methoxyphenyloxy)-4-methoxycarbonyl-5-methylpyrazol, 3-(Cyclopentylmethyloxy)-4-methoxycarbonyl-5-methylpyrazol, 1-Acetyl-3-cyclopentylmethyloxy-4-methoxycarbonyl-5-methylpyrazol, 3-(2-Methylcyclohexyloxy)-4-methoxycarbonyl-5-methylpyrazol.

**Ansprüche für Vertragsstaat:** AT

1. Herbizid, enthaltend ein Pyrazolätherderivat der Formel

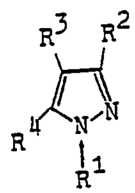

(1)

in welcher $R^1$ Wasserstoff oder $-\underset{\underset{O}{\|}}{C}-R^5$ bedeutet, wobei

$R^5$ Wasserstoff, Alkoxyalkyl, Alkyl oder Halogenalkyl bedeutet,

$R^1$ ferner $-\underset{\underset{O}{\|}}{C}-O-R^6$ bedeutet, wobei

$R^6$ Alkyl oder Phenyl bedeutet, das gegebenenfalls ein- oder mehrfach durch Halogen substituiert ist,

$R^2$ die Gruppe $Y-R^7$ bedeutet, wobei Y Sauerstoff oder Schwefel bedeutet und $R^7$ einen aliphatischen, cycloaliphatischen oder araliphatischen Kohlenwasserstoffrest, Cyclopentylmethyl oder einen Arylrest bedeutet, wobei dieser Arylrest gegebenenfalls ein- oder mehrfach durch Alkyl, Halogen, Halogenalkyl, Alkoxy substituiert ist,

$R^3$ den Methoxycarbonylrest bedeutet und

$R^4$ Wasserstoff oder Methyl bedeutet oder die Salze der Pyrazolätherderivate.

2. Verfahren zur Bekämpfung unerwünschten Pflanzenwuchses, *dadurch gekennzeichnet,* daß man den Boden oder die Pflanzen behandelt mit einem Herbizid gemäß Anspruch 1.

3. Verfahren zur Herstellung eines Pyrazolätherderivates für ein Herbizid gemäß Anspruch 1,

*dadurch gekennzeichnet,* daß man ein Thiokohlensäureesterhydrazid der Formel $R^2$—C—N—$NH_2$
‖ |
S $R^1$

umsetzt mit einer 2-Chlor-1,3-dicarbonylverbindung der Formel $R^4$—CO—CHCl—$R^3$, wobei $R^1$—$R^4$ die im Anspruch 1 genannten Bedeutungen haben, und das so erhaltene Pyrazolderivat gegebenenfalls acyliert.

4. Herbizid gemäß Anspruch 1, enthaltend ein Pyrazolätherderivat, ausgewählt aus der Gruppe, bestehend aus den Verbindungen 3-(2-Methyl-propyloxy)-4-methoxycarbonyl-5-methylpyrazol, 3-Phenyloxy-4-methoxycarbonyl-5-methylpyrazol, 3-(2,2-Dimethylpropyloxy)-4-methoxycarbonyl-5-methylpyrazol, 3-(3-Methoxyphenyloxy)-4-methoxycarbonyl-5-methylpyrazol, 3-(Cyclopentylmethyloxy)-4-methoxycarbonyl-5-methylpyrazol, 1-Acetyl-3-cyclopentylmethyloxy-4-methoxycarbonyl-5-methylpyrazol, 3-(2-Methylcyclohexyloxy)-4-methoxycarbonyl-5-methylpyrazol.

**Claims for the Contracting States :** BE, CH, DE, FR, GB, JT, LU, NL, SE

1. A pyrazole ether derivative of the formula

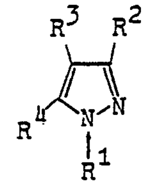

(1)

where $R^1$ denotes hydrogen or —C(=O)—$R^5$, $R^5$ denoting hydrogen,

alkoxyalkyl, alkyl or haloalkyl, $R^1$ further denotes —C(=O)—O—$R^6$, $R^6$ denoting alkyl or phenyl optionally mono- or polysubstituted by halogen, $R^2$ denotes Y—$R^7$, Y denoting oxygen or sulfur and $R^7$ denoting an aliphatic, cycloaliphatic or araliphatic hydrocarbon radical, cyclopentylmethyl or aryl, this aryl being optionally mono- or poly-substituted by alkyl, halogen, haloalkyl or alkoxy, $R^3$ denotes methoxycarbonyl, and $R^4$ denotes hydrogen or methyl, and the salts of the pyrazole ether derivatives.

2. A herbicide containing a pyrazole ether derivative as claimed in claim 1.

3. A herbicide containing a solid or liquid carrier and a pyrazole ether derivative as claimed in claim 1.

4. A process for controlling the growth of unwanted plants *characterized in that* the soil or the plants are treated with a pyrazole ether derivative as claimed in claim 1.

5. A process for preparing a pyrazole ether derivative as claimed in claim 1, *characterized in that* a thiocarbonic acid ester hydrazide of the formula $R^2$—C—N—$NH_2$
‖ |
S $R^1$

is reacted with a 2-chloro-1,3-dicarbonyl compound of the formula $R^4$—CO—CHCl—$R^3$, $R^1$—$R^4$ having the meanings given in claim 1, and the pyrazole derivative thus obtained is optionally acylated.

6. A pyrazole ether derivative selected from the group consisting of the compounds 3-(2-methylpropyloxy)-4-methoxycarbonyl-5-methylpyrazole, 3-phenyloxy-4-methoxycarbonyl-5-methylpyrazole, 3-(2,2-dimethylpropyloxy)-4-methoxycarbonyl-5-methylpyrazole, 3-(3-methoxyphenyloxy)-4-methoxycarbonyl-5-methylpyrazole, 3-(cyclopentylmethyloxy)-4-methoxycarbonyl-5-methylpyrazole, 1-acetyl-3-cyclopentylmethyloxy-4-methoxycarbonyl-5-methylpyrazole and 3-(2-methylcyclohexyloxy)-4-methoxycarbonyl-5-methylpyrazole.

7. A herbicide as claimed in claim 2, containing a pyrazole ether derivative selected from the group consisting of the compounds 3-(2-methylpropyloxy)-4-methoxycarbonyl-5-methylpyrazole, 3-phenyloxy-4-methoxycarbonyl-5-methylpyrazole, 3-(2,2-dimethylpropyloxy)-4-methoxycarbonyl-5-methylpyrazole, 3-(3-methoxyphenyloxy)-4-methoxycarbonyl-5-methylpyrazole, 3-(cyclopentylmethyloxy)-4-methoxycarbonyl-5-methylpyrazole, 1-acetyl-3-cyclopentylmethyloxy-4-methoxycarbonyl-5-methylpyrazole and 3-(2-methylcyclohexyloxy)-4-methoxycarbonyl-5-methylpyrazole.

**Claims for contractingstate :** AT

1. A herbicide containing a pyrazole ether derivative of the formula

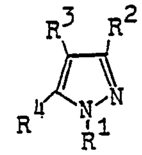

(1)

where $R^1$ denotes hydrogen of $-\underset{\underset{O}{\|}}{C}-R^5$, $R^5$ denoting hydrogen, alkoxyalkyl, alkyl or haloalkyl, $R^1$ further denotes $-C-\underset{\underset{O}{\|}}{O}-R^6$ $R^6$ denoting alkyl or phenyl optionnally mono- or polysubstituted by halogen, $R^2$ denotes $Y-R^7$, Y denoting oxygen or sulfur and $R^7$ denoting an aliphatic, cycloaliphatic or araliphatic hydrocarbon radical, cyclopentylmethyl or aryl, this aryl being optionally mono- or polysubstituted by alkyl, halogen, haloalkyl or alkoxy, $R^3$ denotes methoxycarbonyl, and $R^4$ denotes hydrogen or methyl, or the salts of the pyrazole ether derivatives.

2. A process for controlling the growth of unwanted plants *characterized in that* the soil of the plants are treated with a herbicide as claimed in claim 1.

3. A process for preparing a pyrazole ether derivative for a herbicide as claimed in claim 1, *characterized in that* a thiocarbonic acid ester hydrazide of the formula $R^2-\underset{\underset{S}{\|}}{C}-\underset{\underset{R^1}{|}}{N}-NH_2$ is reacted with a 2-chloro-1,3-dicarbonyl compound of the formula $R^4-CO-CHCl-R^3$, $R^1-R^4$ having the meanings given in claim 1, and the pyrazole derivative thus obtained is optionally acylated.

4. A herbicide as claimed in claim 1, containing a pyrazole ether derivative selected from the group consisting of the compounds 3-(2-methylpropyloxy)-4-methoxycarbonyl-5-methylpyrazole, 3-phenyloxy-4-methoxycarbonyl-5-methylpyrazole, 3-(2,2-dimethylpropyloxy)-4methoxycarbonyl-5-methylpyrazole, 3-(3-methoxypenyloxy)-4-methoxycarbonyl-5-methylpyrazole, 3-(cyclopentylmethyloxy)-4-methoxycarbonyl-5-methylpyrazole, 1-acetyl-3-cyclopentylmethyloxy-4-methoxycarbonyl-5-methylpyrazole and 3-(2-methylcyclohexyloxy)-4-methoxycarbonyl-5-methylpyrazole.

**Revendications pour les Etats contractants :** BE, CH, DE, FR, GB, JT, LU, NL, SE

1. Dérivé d'éther pyrazolique ou ses sels de formule

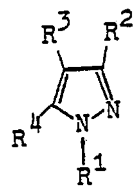

dans laquelle

$R^1$ représente l'hydrogène ou $-\underset{\underset{O}{\|}}{C}-R^5$, $R^5$ représentant hydrogène, alcoxyalkyle, alkyle ou halogènealkyle

$R^1$ représente encore $-\underset{\underset{O}{\|}}{C}-O-R^6$, $R^6$ représentant alkyle ou phényle éventuellement substitué une ou plusieurs fois par un halogène,

$R^2$ représente le groupe $Y-R^7$, Y représentant oxygène ou soufre et $R^7$ représentant un reste — hydrocarbure aliphatique cycloaliphatique ou araliphatique, un reste cyclopentylméthyle ou un reste aryle, ce reste aryle étant éventuellement substitué une ou plusieurs fois par un alkyle, halogène, halogènealkyle ou alcoxy,

$R^3$ représente le reste méthoxycarbonyle et

$R^4$ représente l'hydrogène ou le méthyle.

2. Herbicide contenant un dérivé d'éther pyrazolique selon la revendication 1.

3. Herbicide contenant un support solide ou liquide et un dérivé d'éther pyrazolique selon la revendication 1.

4. Procédé pour lutter contre la croissance indésirable des plantes, caractérisé par le fait que l'on traite le sol ou les plantes avec un dérivé d'éther pyrazolique selon la revendication 1.

5. Procédé de préparation d'un dérivé d'éther pyrazolique selon la revendication 1, caractérisé par le fait qu'on fait réagir un hydrazide d'ester thiocarboxylique de formule $R^2-\underset{\underset{S}{\|}}{C}-\underset{\underset{R^1}{|}}{N}-NH_2$ avec un composé 2-chloro-1,3- dicarbonyle de formule $R^4-CO-CHCl-R^3$, $R^1$ à $R^4$ ayant les significations indiquées dans la revendication 1, et on acyle éventuellement le dérivé pyrazolique ainsi obtenu.

6. Dérivé d'éther pyrazolique selon la revendication 1, choisi dans le groupe constitué par les composés 3-(2-méthylpropyloxy)-4-méthoxycarbonyl-5-méthylpyrazole, 3-phényloxy-4-méthoxycarbonyl-5-méthylpyrazole, 3-(2,2-diméthylpropyloxy)-4-méthoxycarbonyl-5-méthylpyrazole, 3-(3-méthoxyphényloxy)-4-méthoxycarbonyl-5-méthylpyrazole, 3-(cyclopentylméthyloxy)-4-méthoxycarbonyl-5-méthylpyrazole, 1-acétyl-3-cyclopentylméthyloxy-4-méthoxycarbonyl-5-méthylpyrazole, 3-(2-méthylcyclohexyloxy)-4-méthoxycarbonyl-5-méthylpyrazole.

7. Herbicide selon la revendication 2 contenant un dérivé d'éther pyrazolique, choisi dans le groupe constitué des composés 3-(2-méthylpropyloxy)-4-méthoxycarbonyl-5-méthylpyrazole, 3-phényloxy-4-méthoxycarbonyl-5-méthylpyrazole, 3-(2,2-diméthylpropyloxy)-4-méthoxycarbonyl-5-méthylpyrazole, 3-(3-méthoxyphényloxy)-4-méthoxycarbonyl-5-méthylpyrazole, 3-(cyclopentylméthyloxy)-4-méthoxycarbonyl-5-méthylpyrazole, 1-acétyl-3-cyclopentylméthyloxy-4-méthoxycarbonyl-5-méthylpyrazole, 3-(2-méthylcyclohexyloxy)-4-méthoxycarbonyl-5-méthylpyrazole.

**Revendications pour l'Etat contractant :** AT

1. Herbicide contenant un dérivé d'éther pyrazolique ou ses sels, de formule

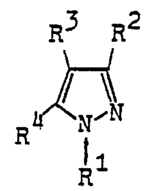

dans laquelle

$R^1$ représente l'hydrogène ou $-\underset{\underset{O}{\|}}{C}-R^5$, $R^5$ représentant hydrogène, alcoxyalkyle, alkyle ou halogènealkyle $R^1$ représente encore $-\underset{\underset{O}{\|}}{C}-O-R^6$, $R^6$ représentant alkyle ou phényle éventuellement substitué une ou plusieurs fois par un halogène,

$R^2$ représente le groupe Y-$R^7$, Y représentant oxygène ou soufre et $R^7$ représentant un reste — hydrocarbure aliphatique, cycloaliphatique ou araliphatique, un reste cyclopentylméthyle ou un reste aryle, ce reste aryle étant éventuellement substitué une ou plusieurs fois par un alkyle, halogène, halogènealkyle ou alcoxy

$R^3$ représente le reste méthoxycarbonyle et

$R^4$ représente l'hydrogène ou le méthyle.

2. Procédé pour lutter contre la croissance indésirable des plantes, caractérisé par le fait que l'on traite le sol ou les plantes avec un herbicide selon la revendication 1.

3. Procédé de préparation d'un dérivé d'éther pyrazolique pour un herbicide selon la revendication 1, caractérisé par le fait qu'on fait réagir un hydrazide d'ester thiocarboxylique de formule $R^2-\underset{\underset{S}{\|}}{C}-\underset{\underset{R^1}{|}}{N}-NH_2$ avec un composé 2-chloro-1,3-dicarbonyle de formule $R^4-CO-CHCl-R^3$, $R^1$ à $R^4$ ayant les significations indiquées dans la revendication 1, et on acyle éventuellement le dérivé pyrazolique ainsi obtenu.

4. Herbicide selon la revendication 1 contenant un dérivé d'éther pyrazolique, choisi dans le groupe constitué des composés 3-(2-méthylpropyloxy)-4-méthoxycarbonyl-5-méthylpyrazole, 3-phényloxy-4-méthoxycarbonyl-5-méthylpyrazole, 3-(2,2-diméthylpropyloxy)-4-méthoxycarbonyl-5-méthyl-pyrazole, 3-(3-méthoxyphényloxy)-4-méthoxycarbonyl-5-méthylpyrazole, 3-(cyclopentylméthyloxy)-4-méthoxycarbonyl-5-méthylpyrazole, 1-acétyl-3-cyclopentylméthyloxy-4-méthoxycarbonyl-5-méthylpyrazole, 3-(2-méthylcyclohexyloxy)-4-méthoxycarbonyl-5-méthylpyrazole.